# FASCICULE DE BREVET EUROPEEN

(11) **EP 2 892 652 B1**
(45) Date de publication et mention de la délivrance du brevet: **27.02.2019**
(21) Numéro de dépôt: 13766617.8
(22) Date de dépôt: 06.09.2013
(51) Int. Cl.: B03C 1/28, G01N 33/543, B03C 1/01

(54) **SYSTEME MICROFLUIDIQUE PRESENTANT UN LIT DE PARTICULES MAGNETIQUES**
MIKROFLUIDISCHES SYSTEM MIT EINEM MAGNETPARTIKELBETT
MICROFLUIDIC SYSTEM HAVING A MAGNETIC PARTICLE BED

(30) Priorité: 07.09.2012 FR 1258396
(43) Date de publication de la demande: 15.07.2015
(73) Titulaire: Viovy, Jean-Louis, 75013 Paris (FR); Tabnaoui, Sanae, 4125 Riehen (CH); Malaquin, Laurent, 91310 Linas (FR); Descroix, Stéphanie, 75014 Paris (FR)
(72) Inventeur: Viovy, Jean-Louis, 75013 Paris (FR); Tabnaoui, Sanae, 4125 Riehen (CH); Malaquin, Laurent, 91310 Linas (FR); Descroix, Stéphanie, 75014 Paris (FR)
(74) Mandataire: Bandpay & Greuter
(86) Numéro de dépôt international: PCT/FR2013/052057
(87) Numéro de publication internationale: WO 2014/037674

(56) Documents cités:
- WO-A1-2011/155489
- WO-A2-2010/041230

## Description

### DOMAINE DE L'INVENTION

La présente invention concerne un système microfluidique présentant un lit de particules magnétiques, ainsi qu'un procédé d'analyse reposant sur l'utilisation de ce système.

### ARRIERE-PLAN TECHNIQUE

Les systèmes microfluidiques permettent de manipuler de faibles volumes de fluide, jusqu'à moins de 1 µL. Ils ont ainsi ouvert la voie à de nouvelles applications en biologie, en chimie ou en physique, impossibles à mener à bien avec des systèmes conventionnels.

Ces systèmes permettent par exemple d'effectuer des analyses à l'échelle de molécules individuelles ou de cellules individuelles et d'effectuer des réactions biochimiques dans des volumes très réduits, augmentant grandement la dynamique et la fiabilité des réactions : les réactions de polymérisation en chaîne (PCR) à l'échelle de la molécule d'ADN individuelle, les technologies de séquençage de nouvelle génération sont des exemples de telles analyses.

A l'échelle macroscopique, il est connu d'effectuer des opérations de purification, extraction et concentration au moyen d'une phase solide, notamment dans le cadre d'applications chromatographiques ou d'immuno-affinité. Mais la mise en oeuvre de microcolonnes de séparation chromatographiques dans des systèmes microfluidiques pose de sérieux problèmes en termes d'homogénéité des microcolonnes; en outre, des pressions élevées sont nécessaires pour opérer la circulation des fluides dans les systèmes microfluidiques, et la micro-fabrication de systèmes de géométries complexes peut être laborieuse.

L'utilisation de particules magnétiques, et notamment super-paramagnétiques, en tant que phase solide dans des systèmes à l'échelle macroscopique, a connu une certaine popularité. En effet, les particules magnétiques liées à un analyte d'intérêt peuvent être retenues par un aimant, tandis que le fluide environnant est éliminé. Les procédés de ce type peuvent être multiplexés, par exemple en utilisant de multiples aimants disposés au fond de plaques de microtitration. De tels systèmes présentent toutefois l'inconvénient des limitations par exemple vis à vis de la vitesse de réaction, de volumes d'élution nécessaires relativement élevés, et d'efficacité de mélange et de rinçage médiocre.

Utiliser des particules super-paramagnétiques dans des systèmes microfluidiques peut permettre de résoudre ces problèmes : en effet le rapport surface / volume est alors élevé, les possibilités de fonctionnalisation des surfaces sont nombreuses, tandis que les propriétés magnétiques des particules permettent une manipulation aisée sans contact, et permettent la formation de structures compactes telles que des micro-colonnes.

Un défi posé par les méthodes de détection sur puces est celui de la concentration des analytes dans le volume d'analyse, ou sur la surface de détection, jusqu'à un niveau qui doit être suffisamment élevée vis-à-vis du seuil de détection. Il est donc souhaitable de concentrer les échantillons avant détection, ce qui est délicat à réaliser. Ce défi est particulièrement important dans le cas des applications de diagnostic, dans lesquelles des marqueurs biologiques peuvent être présents à très faible concentration. Pour un système microfluidique typique fonctionnant avec un volume d'échantillon de 1 µL ou moins, une étape de pré-concentration à partir d'un volume plus grand (par exemple de plusieurs mL) peut être nécessaire.

Le document WO 98/23379 décrit un dispositif de séparation de particules ou de molécules par migration à travers un ferrofluide. Un champ magnétique est appliqué perpendiculairement à la direction de déplacement des espèces à séparer, pour créer des zones riches et pauvres en particules magnétiques.

Le document EP 1331035 décrit un appareil pour retenir des particules magnétiques dans une cellule de circulation de fluide. La cellule est placée entre les pôles d'un aimant, et de forts gradients locaux de champ magnétiques sont générés au moyen de microstructures présentes à la surface des pôles de l'aimant. Ce sont ces forts gradients qui immobilisent les particules magnétiques.

Le document EP 1974821 décrit un système dans lequel les particules magnétiques peuvent être déplacées le long d'un canal par une succession d'électroaimants se faisant face de part et d'autre des parois latérales du canal.

Le document US 7,309,439 décrit un dispositif permettant de transporter des particules magnétiques dans un tube capillaire, au moyen de dispositifs magnétiques placés autour du tube.

L'article de Beyor et al. dans Biomed. Microdevices 10:909-917 (2008) décrit un système utilisant des particules magnétiques pour détecter des agents pathogènes sur puce. Un aimant mobile est placé sous un micro-canal comportant des bifurcations, ce qui crée un barrage compact de particules pouvant être déplacé.

L'article de Gijs et al. dans Chem. Rev. 110:1518 (2010) est une revue des applications microfluidiques des particules magnétiques pour l'analyse biologique et la catalyse. En particulier, il est exposé que les particules magnétiques peuvent être retenues et manipulées dans des microsystèmes au moyen d'aimants ou d'électroaimants fixes ou mobiles placés d'un côté d'un canal (en dessous) ou se faisant face de part et d'autre du canal (au-dessus et en dessous).

Le document WO 2010/041231 décrit encore un système dans lequel des particules magnétiques sont immobilisées au moyen d'un champ magnétique transversal à la direction du flux.

Le document WO 2010/041230 décrit un dispositif microfluidique selon le préambule de la revendication 1 pour la détection d'analytes. Le dispositif comprend un micro-canal et des aimants disposés de part et d'autre du micro-canal et orientés de telle sorte qu'un champ magnétique essentiellement colinéaire à la direction du flux dans le micro-canal est généré. Ce système permet également de créer un bouchon de particules magnétiques dans une zone du micro-canal. Il n'est pas approprié pour une utilisation à des débits relativement élevés.

Enfin, on peut noter que des systèmes proposant une circulation de fluide à travers des combinaisons de particules magnétiques et non magnétiques ont été proposés.

Ainsi, l'article de Seibert et al. dans Biotechnol. Prog. 14:749-755 (1998) décrit un système macroscopique dans lequel une série de bobines annulaires sont placées autour d'un tube contenant un lit fluidisé pour fermentation, contenant des particules magnétiques et non magnétiques. Les particules magnétiques réduisent les effets de mélange dans le lit.

L'article de Tong et al. dans Biotechnol. Prog. 19:1721-1727 (2003) décrit un autre système macroscopique comprenant une bobine unique autour d'un lit fluidisé créant un champ transverse, qui est utilisé pour augmenter la compacité du lit.

Cependant, ces deux derniers systèmes ne permettent pas de traiter de petits volumes de fluide.

En résumé de ce qui précède, l'état de la technique propose deux types de systèmes microfluidiques comportant des particules magnétiques. Dans le premier type, les particules sont organisées en chaînes statiques de faible densité. Le débit de fluide dans ce type de systèmes peut être relativement élevé, de par la faible densité des particules magnétiques. Mais le temps de transport des espèces d'intérêt vers les particules magnétiques est élevé du fait des distances importantes à parcourir. Dans le deuxième type, les particules sont organisées en blocs compacts, avec généralement des zones de circulation de fluide dans le voisinage des blocs compacts. Ces blocs compacts forment des bouchons vis-à-vis de l'écoulement, de sorte que le débit est limité.

Il existe donc un besoin de surmonter les inconvénients des systèmes de l'état de la technique, et notamment de disposer d'un système microfluidique fonctionnant avec un débit relativement élevé, tout en assurant un bon contact entre les espèces d'intérêt et les particules magnétiques, c'est-à-dire un faible temps de diffusion des espèces d'intérêt vers les particules magnétiques.

### RESUME DE L'INVENTION

Un premier objet de l'invention concerne un système microfluidique comprenant :
- au moins un canal pour l'écoulement de fluide présentant une entrée, une sortie, et un axe longitudinal s'étendant entre l'entrée et la sortie, ledit canal comprenant une zone de capture, et la section du canal orthogonale à l'axe longitudinal du canal étant croissante dans la zone de capture, de l'entrée vers la sortie du canal ; et
- des moyens d'application d'un champ magnétique présentant une intensité décroissante dans la zone de capture du canal, de l'entrée vers la sortie du canal.

Selon un mode de réalisation, le champ magnétique appliqué dans la zone de capture est essentiellement parallèle à l'axe longitudinal du canal.

Selon un mode de réalisation, les moyens d'application du champ magnétique comprennent ou consistent en un aimant disposé à l'extérieur du canal, de préférence du côté de l'entrée de celui-ci.

Selon un mode de réalisation, la zone de capture du canal comprend des particules magnétiques, de préférence des particules super-paramagnétiques, la fraction volumique de ces particules dans la zone de capture du canal étant de préférence de 0,01 à 0,3.

Selon un mode de réalisation, la sortie du canal est connectée à une conduite secondaire dont la résistance hydraulique est supérieure à la résistance hydraulique du canal, de préférence d'un facteur supérieur ou égal à 2, ou à 5 ou à 10.

Selon un mode de réalisation, le système comprend des moyens de déplacement de fluide pour effectuer l'écoulement de fluide de l'entrée vers la sortie du canal, et optionnellement un dispositif adapté à générer des oscillations de fluide dans le canal, de préférence choisi parmi les transducteurs soniques, ultrasoniques, ou piézoélectriques, les éléments vibrants, les haut-parleurs et les pistons oscillants.

Un deuxième objet de l'invention concerne un procédé de traitement d'un échantillon, comprenant une étape d'écoulement de fluide dans un canal présentant une zone de capture, la vitesse d'écoulement du fluide étant décroissante dans la zone de capture, et un champ magnétique étant appliqué dans la zone de capture, présentant une intensité décroissante dans le sens d'écoulement du fluide.

Le procédé de traitement d'un échantillon peut être notamment un procédé d'analyse d'échantillon, ou par exemple un procédé de synthèse, de réaction chimique, de modification, de séparation de l'échantillon.

Selon un mode de réalisation, lors de l'étape d'écoulement de fluide, le champ magnétique appliqué est essentiellement parallèle à la direction moyenne d'écoulement du fluide dans la zone de capture.

Selon un mode de réalisation, lors de l'étape d'écoulement de fluide, la zone de capture contient des particules magnétiques, de préférence super-paramagnétiques, et dans lequel le fluide s'écoulant dans le canal contient des analytes ; le procédé comprenant optionnellement une étape supplémentaire de détection d'analytes et / ou une étape supplémentaire de collecte d'analytes en aval du canal.

Selon un mode de réalisation, le procédé comprend la mise en oeuvre d'une réaction et / ou séparation chimique, biochimique et / ou biologique, de préférence choisie parmi les réactions catalytiques, les hybridations, les réactions électrochimiques, les réactions enzymatiques, les immunodosages, les séparations chromatographiques, les réactions de chimioluminescence, les captures immunologiques, les captures par affinité, les élutions, les purifications, les concentrations, les extractions et combinaisons de celles-ci.

Selon un mode de réalisation, le procédé de l'invention est mis en oeuvre dans le système microfluidique de l'invention.

La présente invention permet de surmonter les inconvénients de l'état de la technique. Elle fournit plus particulièrement un système microfluidique susceptible de fonctionner avec un débit relativement élevé, tout en assurant un bon contact entre les espèces d'intérêt et les particules magnétiques, c'est-à-dire un faible temps de diffusion des espèces d'intérêt vers les particules magnétiques.

Cela est accompli grâce à la combinaison d'une géométrie de canal particulière, avec une section croissante dans la direction de l'écoulement du fluide, et de l'application d'un champ magnétique décroissant selon cette direction de l'écoulement du fluide. De la sorte, il est possible de retenir des particules magnétiques dans une zone d'intérêt du micro-canal par équilibre entre les forces magnétiques et les forces hydrodynamiques appliquées.

Ainsi, on dispose d'une région dans laquelle les particules magnétiques à la fois présentent une densité souhaitée, et de préférence ne sont pas organisées de façon compacte, de façon à permettre un écoulement de fluide avec un débit relativement rapide, et sont réparties de manière relativement homogène dans le fluide, assurant ainsi un transport optimal (et un temps de diffusion minimal) des analytes du fluide vers les particules magnétiques.

L'invention fournit ainsi un lit fluidisé de particules magnétiques dans un micro-canal.

Dans un certain nombre d'applications, la sensibilité des systèmes microfluidiques selon l'invention est supérieure à celle des systèmes de l'état de la technique.

L'invention est particulièrement utile dans le cas où une étape de concentration d'analytes préalable à la détection est nécessaire (par exemple à partir d'un volume de quelques mL jusqu'à un volume de quelques µL, voire de moins de 1 µL).

Outre les caractéristiques principales de l'invention énumérées ci-dessus, le procédé et le système selon l'invention peuvent en outre présenter l'une quelconque ou plusieurs des autres caractéristiques qui suivent.

Selon un mode de réalisation, le champ magnétique dans la zone de capture du canal ne présente aucun pic ou maximum local d'intensité.

Selon un mode de réalisation, l'intensité du champ magnétique appliqué dans la zone de capture du canal varie de manière continue, par exemple linéairement, dans la direction de l'écoulement.

Selon un mode de réalisation, l'intensité du champ magnétique appliqué dans la zone de capture du canal varie de manière continue, par exemple linéairement, selon l'axe longitudinal du canal.

Selon un mode de réalisation, la vitesse moyenne du fluide (intégrée sur une section transversale du canal, orthogonale à l'axe longitudinal) dans la zone de capture du canal varie de manière continue, dans la direction de l'écoulement (ou selon l'axe longitudinal du canal).

Selon un mode de réalisation, l'intensité du champ magnétique est supérieure à l'entrée du canal par rapport à la sortie du canal.

Selon un mode de réalisation, l'intensité du champ magnétique est la plus grande dans la partie de la zone de capture qui présente la plus petite section transversale.

Selon un mode de réalisation, l'intensité du champ magnétique est la plus petite dans la partie de la zone de capture qui présente la plus grande section transversale.

Selon un mode de réalisation, la section transversale de la zone de capture croît continûment de l'entrée vers la sortie, et l'intensité du champ magnétique de la zone de capture décroît continûment de l'entrée vers la sortie

Selon un mode de réalisation, la zone de capture comporte des particules magnétiques, au moins 50 %, de préférence au moins 70 % ou au moins 90 % des particules n'étant pas empilées de façon compacte.

Selon un mode de réalisation, la zone de capture comporte des particules magnétiques piégées dans la zone de capture, les particules magnétiques subissant une recirculation continue lors de l'écoulement d'un fluide de l'entrée vers la sortie du canal.

Selon un mode de réalisation, le système selon l'invention comprend un dispositif de contrôle de la différence de pression entre l'entrée et la sortie du système et un débitmètre pour mesurer le débit de fluide entrant dans ou sortant de la zone de capture, le dispositif de contrôle de la pression étant adapté à régler la pression à l'entrée du canal, ou à régler la différence de pression entre l'entrée et la sortie du canal, en fonction du débit de fluide mesuré par le débitmètre.

Selon un mode de réalisation, le procédé de l'invention comprend une étape d'application d'une pression prédéfinie à l'entrée du canal, tout en maintenant les particules magnétiques dans la zone de capture dans un état d'empilement compact.

Un troisième objet de l'invention est un système microfluidique comprenant un canal comprenant une zone de capture soumise à un champ magnétique présentant un gradient d'intensité le long de la zone de capture, une source de fluide connectée à une entrée de la zone de capture, et une conduite secondaire connectée au canal en amont ou en aval de la zone de capture et présentant une résistance hydraulique supérieure à la résistance hydraulique de la zone de capture du canal (en l'absence de particules magnétiques), de préférence d'un facteur d'au moins deux fois, ou d'au moins cinq, ou d'au moins dix, et par exemple de dix à cent fois supérieure.

Selon un mode de réalisation de ce troisième objet de l'invention, la zone de capture du canal s'élargit selon la direction principale de l'écoulement de fluide dans le canal.

Selon un mode de réalisation de ce troisième objet de l'invention, le système comporte un équipement magnétique susceptible de produire un champ magnétique orienté selon l'axe longitudinal du canal dans la zone de capture, l'intensité du champ magnétique décroissant (de préférence continûment) dans la zone de capture.

Un quatrième objet de l'invention est un système microfluidique comprenant :
- au moins un canal pour l'écoulement de fluide présentant une entrée, une sortie, et un axe longitudinal s'étendant entre l'entrée et la sortie, ledit canal présentant une zone de capture ;
- des moyens d'application d'un champ magnétique ;
dans lequel, dans la zone de capture du canal :
- la section du canal orthogonale à l'axe longitudinal croît de l'entrée vers la sortie du canal ;
- le champ magnétique appliqué est essentiellement parallèle à l'axe longitudinal du canal.

Associé à celui-ci est un procédé d'analyse, comprenant une étape d'écoulement de fluide dans un canal présentant une zone de capture, la vitesse d'écoulement du fluide étant décroissante dans la zone de capture, et un champ magnétique étant appliqué dans la zone de capture, essentiellement parallèlement à la direction d'écoulement du fluide.

Un cinquième objet de l'invention est un système microfluidique comprenant un canal pourvu d'une entrée et d'une sortie, le canal s'élargissant à partir de l'entrée pour former une zone de capture, et un équipement de génération de champ magnétique étant disposé à l'extérieur du canal, du côté de l'entrée du canal (c'est-à-dire que l'entrée du canal est le point du canal le plus proche de l'équipement).

Un sixième objet de l'invention est un système microfluidique comprenant un lit fluidisé de particules magnétiques stabilisé par un champ magnétique dans une zone de capture d'un canal, ledit champ magnétique décroissant de façon essentiellement monotone d'une extrémité à l'autre de la zone de capture.

Dans certains modes de réalisation, chacun de ces troisième, quatrième, cinquième et sixième objets de l'invention peut présenter en outre les caractéristiques décrites ci-dessus en rapport avec le premier objet et le deuxième objet de l'invention.

Un autre objet de l'invention est un procédé comprenant les étapes de :
- circulation d'un fluide contenant des particules magnétiques dans un canal comprenant une zone de capture présentant une région d'expansion, un champ magnétique étant appliqué dans la zone de capture, avec une intensité décroissante dans le sens de l'expansion du canal, de sorte à retenir les particules magnétiques dans la zone de capture ;
- circulation d'un fluide contenant des analytes dans le canal, en présence du champ magnétique, à un débit tel que les particules magnétiques sont en mouvement mais restent dans la zone de capture ;
- éventuellement, interaction directe ou indirecte des analytes avec les particules magnétiques retenues.

Avantageusement, ce procédé est un mode de réalisation particulier du procédé d'analyse selon l'invention décrit ci-dessus ou mis en oeuvre avec un système selon l'invention.

Selon un mode de réalisation de l'un quelconque des procédés selon l'invention, on prévoit les étapes successives suivantes :
- fourniture d'un système microfluidique selon l'invention ;
- circulation dans le système microfluidique d'un premier fluide contenant des particules magnétiques portant des ligands, ou d'une combinaison de particules magnétiques et d'autres objets colloïdaux portant des ligands, le champ magnétique étant activé ;
- optionnellement, rinçage ;
- circulation dans le système microfluidique d'un deuxième fluide contenant des analytes ;
- optionnellement, rinçage ;
- élution des analytes éventuellement liés aux ligands et retenus dans la zone de capture du canal par la circulation d'un troisième fluide dans le système mircrofluidique.

Selon un mode de réalisation de l'un quelconque des procédés selon l'invention, on prévoit les étapes successives suivantes :
- fourniture d'un système microfluidique selon l'invention ;
- circulation dans le système microfluidique d'un premier fluide contenant des particules magnétiques portant des ligands, ou d'une combinaison de particules magnétiques et d'autres objets colloïdaux portant des ligands, le champ magnétique étant activé ;
- optionnellement, rinçage ;
- circulation dans le système microfluidique d'un deuxième fluide contenant des analytes ;
- collecte des analytes à une sortie du système microfluidique ;
- optionnellement, détection des analytes collectés, ou traitement supplémentaire des analytes collectés.

Les deux modes de réalisation précédents peuvent être combinés, avec une liaison d'analyte puis une élution et une détection.

Selon un mode de réalisation de l'un quelconque des procédés selon l'invention, on prévoit les étapes successives suivantes :
- fourniture d'un système microfluidique selon l'invention ;
- circulation dans le système microfluidique d'un premier fluide contenant des particules magnétiques portant des ligands, ou d'une combinaison de particules magnétiques et d'autres objets colloïdaux portant des ligands, le champ magnétique étant activé ;
- optionnellement, rinçage ;
- circulation dans le système microfluidique d'un deuxième fluide contenant des analytes ;
- optionnellement, rinçage ;
- optionnellement, circulation d'un troisième fluide dans le système microfluidique, contenant de préférence des ligands supplémentaires ;
- optionnellement, rinçage ;
- injection dans le système microfluidique d'un substrat ;
- détection des résultats d'une réaction dudit substrat.

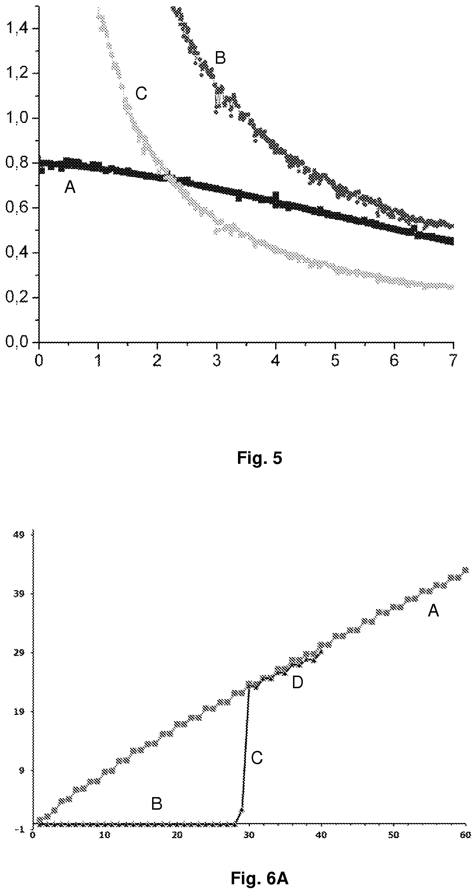

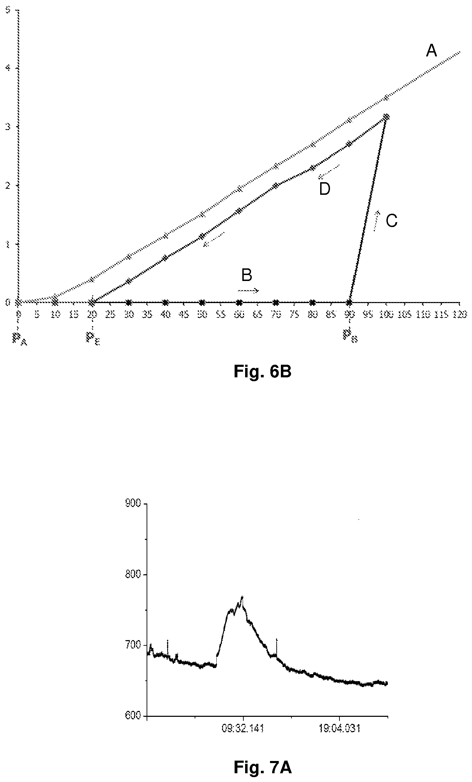

Comme il sera montré plus en détail dans la description de modes de réalisation, et dans certains exemples, notamment en relation avec les **figures 6A** **et** **6B**, la possibilité de contrôler le passage d'un état compact à un état dense non compact confère aussi aux dispositifs selon l'invention des caractéristiques hydrodynamiques originales, notamment en termes de comportements non-linéaires, à seuil, ou encore la possibilité d'obtenir une perte de charge indépendante du débit. Ces dispositifs peuvent donc être avantageux comme dispositifs permettant de contrôler, d'arrêter, de démarrer, de modifier ou de réguler des écoulements ou des pressions au sein d'un système microfluidique. En effet, comme montré dans les exemples, dans certains modes de réalisation, le canal, contenant des particules magnétiques en présence d'un champ magnétique, peut transiter entre un état de forte résistance hydrodynamique et un état de faible résistance hydrodynamique, dans lequel ladite résistance hydrodynamique varie peu en fonction du débit.

Ainsi, l'invention a aussi pour objet dans l'un de ses aspects un dispositif de contrôle d'écoulement ou de pression dans un système microfluidique, caractérisé en ce qu'il comporte :
- au moins un canal pour l'écoulement de fluide présentant une entrée, une sortie, et un axe longitudinal s'étendant entre l'entrée et la sortie, ledit canal comprenant une zone de capture, et la section du canal orthogonale à l'axe longitudinal du canal étant croissante dans la zone de capture, de l'entrée vers la sortie du canal ;
- des particules magnétiques dans la zone de capture du canal ; et
- des moyens d'application d'un champ magnétique présentant une intensité décroissante dans la zone de capture du canal, de l'entrée vers la sortie du canal.

### BREVE DESCRIPTION DES FIGURES

La **figure 1** représente de manière schématique un mode de réalisation du système microfluidique selon l'invention.
Les **figures 2A à 2P** représentent de manière schématique diverses formes possibles pour le canal d'un système microfluidique selon l'invention.
La **figure 3** représente un profil de champ magnétique obtenu dans un mode de réalisation de l'invention (voir exemple 1). La silhouette du canal est représentée en superposition de la représentation du champ magnétique. La distance selon l'axe longitudinal du canal est représentée en abscisse (en µm) et la distance selon la largeur du canal est représentée en ordonnée (en µm). Les flèches représentent les vecteurs du champ magnétique en chaque point, et la teinte du fond représente l'intensité du champ magnétique, selon l'échelle figurant à droite du diagramme (en Tesla).
La **figure 4** représente un champ hydrodynamique obtenu dans un mode de réalisation de l'invention (voir exemple 1). Les axes ont la même signification que pour la **figure 3**. Les flèches représentent la vitesse du fluide en différents points du canal.
La **figure 5** représente les forces magnétiques et hydrodynamiques exercées sur des particules magnétiques dans un mode de réalisation de l'invention (voir exemple 1). En abscisse figure la distance selon l'axe longitudinal du canal (en mm) et en ordonnée figure la valeur de force en 10⁻¹⁰ N.
Les **figures 6A** et **6B** représentent le débit en µL/min (en ordonnée) en fonction de la pression en mbar (en abscisse) dans un mode de réalisation de l'invention (voir exemple 2), respectivement sans conduite secondaire de résistance hydraulique élevée en aval du canal principal, et avec une telle conduite secondaire.
Les **figures 7A** à **7C** représentent des mesures de fluorescence obtenues selon un mode de réalisation de l'invention (voir l'exemple 3). Le temps d'élution figure en abscisse en minutes, et l'intensité de fluorescence figure en ordonnée en unités arbitraires.
La **figure 8** représente le débit en µL/min (en ordonnée) en fonction de la pression en mbar (en abscisse) dans un mode de réalisation de l'invention (voir exemple 4).
La **figure 9** représente de manière schématique un détail d'un mode de réalisation du système microfluidique selon l'invention.

### DESCRIPTION DE MODES DE REALISATION DE L'INVENTION

L'invention est maintenant décrite plus en détail et de façon non limitative dans la description qui suit.

### Architecture du système microfluidique

L'invention concerne un système microfluidique.

Par système microfluidique, on entend de préférence un système comprenant une ou plusieurs microstructures sur la surface d'un substrat, qui constituent des éléments adaptés pour contenir et / ou diriger des fluides. Ces microstructures ont au moins une dimension qui est inférieure à 5 mm, de préférence inférieure à 1 mm, et de manière plus particulièrement préférée inférieure à 500 µm. Dans certains cas, ces microstructures peuvent avoir au moins une dimension inférieure à 200 µm, ou à 100 µm, ou à 50 µm, ou à 20 µm, ou à 10 µm, ou à 5 µm, ou à 2 µm ou à 1 µm.

Ces microstructures peuvent comporter des volumes fermés ou dans certains cas présenter une surface ouverte.

On désigne par canaux (ou micro-canaux) des microstructures adaptées pour la circulation / l'écoulement de fluides. Ils sont le plus souvent fermés sur l'ensemble du parcours des fluides.

Le substrat est de préférence une plaque ou plaquette. Le substrat est de préférence essentiellement rigide, ce qui signifie qu'il peut être manipulé et fixé pour être maintenu immobile, vis-à-vis d'un détecteur par exemple.

Il peut être fait de verre, silicium, céramique, métal ou matériau polymérique / plastique. Le substrat peut être recouvert d'un couvercle de même nature, ou d'un matériau flexible, tel qu'un élastomère silicone, par exemple du polydiméthylsiloxane.

Alternativement, l'ensemble du substrat et du couvercle peuvent être en un matériau flexible, tel qu'un élastomère silicone, par exemple du polydiméthylsiloxane.

La fabrication des microstructures du système microfluidique peut être basée sur des techniques de microfabrication, telles que les techniques de dépôt de film, de photolithographie, de gravure (chimique ou plasma), de thermoformage, de moulage, de moulage par injection, et de collage. Le dépôt de film peut être effectué par centrifugation, par oxydation thermique, par dépôt chimique ou physique en phase vapeur (CVD et PVD), par CVD à basse pression, par CVD améliorée par plasma, par pulvérisation...

Le système microfluidique peut être ou comprendre un laboratoire sur puce.

Le système microfluidique peut comporter un réseau de canaux, c'est-à-dire une pluralité de canaux disposés entre le substrat et son couvercle, ou intégralement entourés par le substrat, et qui sont en communication fluidique soit entre eux, soit avec une ou plusieurs sources de fluide extérieures au système.

Le système microfluidique peut également comporter une série de canaux, c'est-à-dire un ensemble d'une pluralité de canaux non connectés, ou de réseaux de canaux non connectés, sur le même substrat.

Le système microfluidique peut être relié à des réservoirs de fluides ou d'échantillons et autres dispositifs annexes par des tubes ou tuyaux ou connecteurs (par exemple en Y ou en X), afin d'amener des fluides vers, ou de collecter les fluides issus du système. Alternativement, ces tubes ou tuyaux et éventuellement les réservoirs et autres dispositifs annexes peuvent être considérés comme faisant partie du système microfluidique.

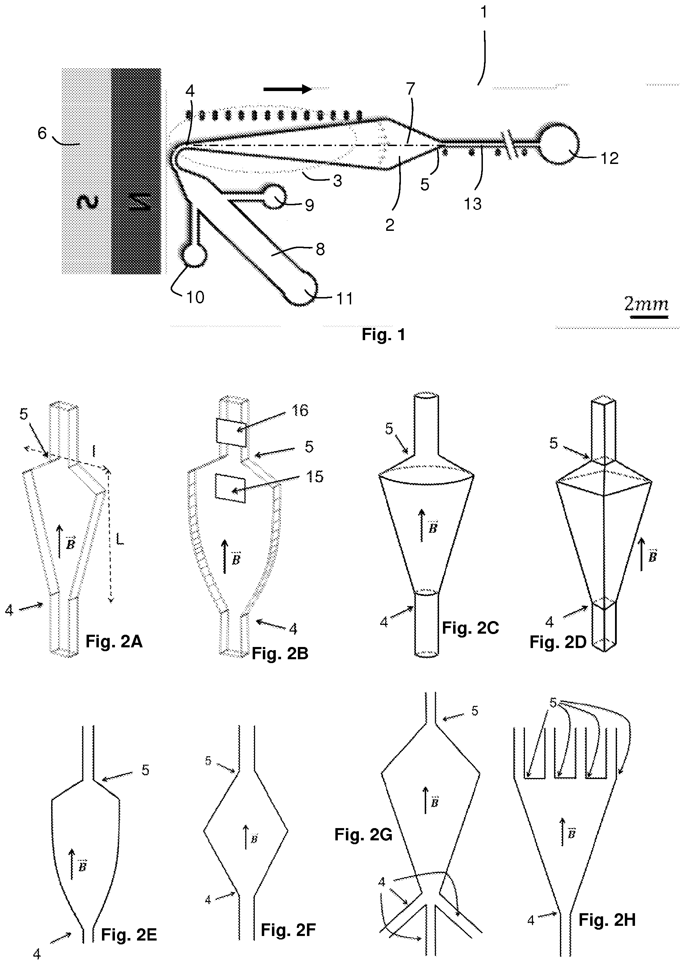

En faisant référence à la **figure 1**, un système microfluidique 1 selon l'invention comporte au moins un canal 2 pour l'écoulement d'un fluide avec une entrée 4 et une sortie 5, ainsi que des moyens d'application de champ magnétique 6.

Il peut se présenter sous la forme d'un ensemble comprenant d'une part le dispositif microfluidique (substrat microstructuré comportant le canal, et les tubulures, réservoirs et autres éléments en connexion fluidique avec celui-ci) et d'autre part les moyens d'application de champ magnétique, non nécessairement fixés ou liés au dispositif microfluidique.

Les termes d'entrée et de sortie sont choisis en référence au sens majoritaire de l'écoulement d'un fluide contenant des analytes (en présence de particules magnétiques dans la zone de capture et de champ magnétique, comme décrit ci-dessous). Pour les besoins de certains protocoles, on peut toutefois être amené, de façon transitoire, à faire circuler certains fluides dans le sens opposé (de la sortie vers l'entrée), ou entre des entrées et sorties différentes de celles utilisées pour la circulation de l'échantillon contenant les analytes, par exemple pour des opérations de rinçage, tout en restant dans le cadre de l'invention.

Le choix du matériau pour former le canal est effectué en fonction de la nature des fluides à transporter, de la forme du canal et des autres éléments du système microfluidique, du coût, de la facilité de production... Le canal peut notamment être fabriqué en verre, en un autre solide non magnétique tel que de la céramique, ou en polymère. Le polymère peut être un élastomère, tel que le polydiméthylsiloxane, ou un polymère fluoré tel que ceux connus sous le nom « Dyneon ». On peut aussi employer un polymère thermoplastique tel qu'un polymère ou copolymère oléfine, notamment oléfine cyclique, polycarbonate, polyméthylméthacrylate, polystyrène, polyéthylène téréphtalate. Les polymères transparents sont préférés, en combinaison éventuellement avec le verre.

Le canal peut également comporter une pluralité d'entrées et / ou une pluralité de sorties, dans l'hypothèse où il s'agit d'un canal ramifié.

En faisant à nouveau référence à la **figure 1**, le canal 2 présente une zone d'intérêt appelée zone de capture 3, qui est adaptée pour contenir un lit de particules magnétiques, et notamment un lit fluidisé (ou l'analogue d'un lit fluidisé à l'échelle d'un système microfluidique) de particules magnétiques.

Le canal 1 présente une forme généralement élongée, avec un axe longitudinal 7 entre l'entrée 4 et la sortie 5.

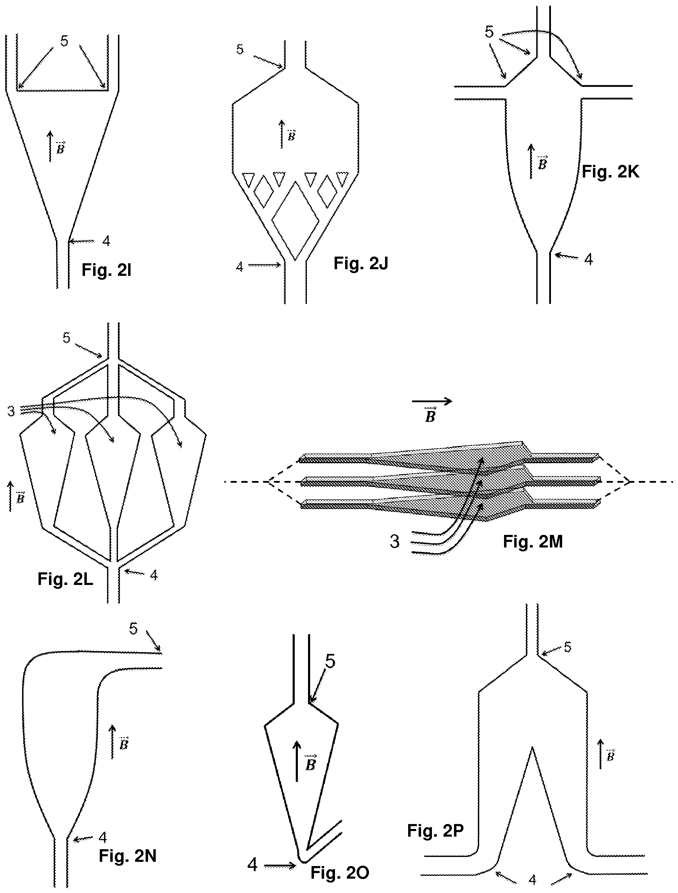

L'axe longitudinal 7 est généralement une droite (au moins dans la zone de capture 3 du canal 2), comme illustré par exemple sur la **figure 1**, mais il peut dans certains cas être composé de segments de droites ou être courbe dans l'hypothèse où le canal comporte des coudes ou virages ou changements de direction (voir par exemple la **figure 2N**).

L'axe longitudinal 7 du canal 2 correspond généralement à la direction moyenne d'écoulement du fluide dans le canal (pouvant être définie comme la direction du vecteur vitesse moyen du fluide dans le canal, en mode d'écoulement non-turbulent).

L'axe longitudinal 7 peut être un axe de symétrie du canal 2, ou au moins de la partie du canal 2 formant la zone de capture 3 ; ou, alternativement, il peut ne pas être un axe de symétrie.

On définit la section transversale du canal comme étant la section orthogonale à l'axe longitudinal 7 du canal 2.

Il est préféré que la section transversale du canal 2 soit supérieure ou égale (de préférence supérieure) en tout point à la section transversale de l'entrée 4 du canal ; et / ou que la section transversale du canal 2 soit supérieure ou égale (de préférence supérieure) en tout point à la section transversale à la sortie 5 du canal 2.

Selon l'invention, la zone de capture 3 a la particularité de s'élargir le long de l'axe longitudinal 7, dans le sens de l'écoulement, c'est-à-dire que la section transversale du canal 2 croît le long de l'axe longitudinal 7 de l'entrée 4 vers la sortie 5 du canal 2 (c'est-à-dire dans le sens de l'écoulement).

Par exemple, le canal peut avoir une forme conique selon son axe longitudinal. Alternativement, et de manière préférée pour une plus grande simplicité de fabrication, le canal peut avoir une section transversale rectangulaire, présentant une hauteur (ou épaisseur, dans la direction perpendiculaire au plan du substrat) et une largeur, la largeur étant croissante dans le sens de l'écoulement (la hauteur restant constante), ou la hauteur étant croissante dans le sens de l'écoulement (la largeur restant constante), ou la largeur et la hauteur étant croissantes dans le sens de l'écoulement.

Le canal a avantageusement une hauteur (épaisseur) constante pour plus de simplicité de fabrication.

De manière générale, le canal peut avoir une section transversale circulaire, ovale, triangulaire, carrée, rectangulaire ou autre (y compris différentes formes à différentes positions le long de l'axe longitudinal), et il peut constituer un volume fermé ou être ouvert sur un côté vers l'environnement externe (le côté supérieur), et ce sur toute la longueur du canal ou seulement une partie de celle-ci. Le canal peut également, de préférence, être fermé, à l'exception de l'entrée et de la sortie.

Le canal peut être un canal capillaire.

La croissance de la section transversale est de préférence continue, et par exemple linéaire.

En aval de la zone de capture du canal, le canal peut comporter une zone aval, qui est donc située entre la zone de capture et la sortie. Cette zone aval peut avoir une section transversale constante ou croissante, mais également, de préférence, une section transversale décroissante vers la sortie, afin de fournir la transition nécessaire vers la sortie qui présente généralement une dimension réduite.

Préférentiellement la zone de capture a une forme généralement allongée. Préférentiellement, l'ensemble du canal a une forme généralement allongée.

Préférentiellement, la longueur du canal, et / ou la longueur de la zone de capture, est supérieure à la dimension maximale du canal dans sa section transversale, et notamment d'un facteur d'au moins 2, ou d'au moins 3 ou d'au moins 5, et pouvant aller jusqu'à 20, 100 ou 500.

La zone de capture peut être dans certains cas ramifiée, auquel cas la section transversale est constituée par la somme des sections transversales des différentes ramifications.

La dimension maximale de la section transversale, peut être par exemple, selon les modes de réalisation, inférieure ou égale à 5 mm, ou à 2 mm, ou à 1 mm, ou à 500 µm, ou à 200 µm, ou à 100 µm, ou à 60 µm, ou à 50 µm, ou à 40 µm, ou à 30 µm, ou à 20 µm, ou à 10 µm, ou à 3 µm, ou à 1 µm, ou à 300 nm, ou à 100 nm, ou à 30 nm, ou à 10 nm.

En outre, le rapport de la longueur de la zone de capture (dimension selon l'axe longitudinal) sur la dimension maximale de la section transversale peut par exemple valoir de 1 à 500, et préférentiellement de 2 à 50, plus particulièrement de 3 à 5, de 5 à 20 ou plus rarement de 20 à 50.

La hauteur ou épaisseur du canal peut aller en général de 1 µm à 5 mm, de préférence de 10 µm à 100 µm ou de 100 µm à 1 mm.

La zone de capture du canal peut avoir un volume allant jusqu'à 10 mL. Il est toutefois préféré qu'elle présente un faible volume, par exemple de 1 mL à 10 mL, ou de 100 µL à 1 mL, ou de 10 µL ou 100 µL, ou de 1 µL à 10 µL, ou de 100 nL à 1 µL, ou de 10 nL à 100 nL, ou même de 1 nL à 10 nL. Les volumes inférieurs à 10 µL sont préférés.

Il peut être approprié que le canal, et notamment sa zone de capture, ou une zone du canal situé en aval de la zone de capture, soit fermé sur l'un de ses côtés par un matériau transparent présentant une épaisseur compatible avec une observation microscopique à haute résolution, formant une « fenêtre ». L'épaisseur de la fenêtre est de préférence inférieure à 500 µm, notamment inférieure à 200 µm.

Les **figures 2A à 2P** illustrent diverses variantes pour la forme du canal décrit ci-dessus, l'entrée (ou les entrées) étant référencée(s) 4 et la sortie (ou les sorties) étant référencée(s) 5. La direction générale du champ magnétique est illustrée par un vecteur sur les schémas.

La **figure 2A** montre un canal avec une seule entrée 4 et une seule sortie 5, une épaisseur constante, une longueur L, et une largeur I qui augmente linéairement depuis un minimum en entrée 4, jusqu'à un maximum (inférieur à la longueur L), puis diminue linéairement jusqu'à la sortie 5. La forme globale du canal est donc celle d'un losange asymétrique. La zone de capture 3 est située entre l'entrée 4 et la zone du canal de largeur maximale.

La **figure 2B** montre une variante avec une largeur qui varie de manière non-linéaire. En outre, une fenêtre 15 (telle que décrite ci-dessus) est ménagée sur une surface supérieure ou inférieure du canal, et / ou une fenêtre 16 est ménagée en aval du canal.

La **figure 2C** montre une variante dans laquelle le canal présente un élargissement tridimensionnel, en forme de cône.

La **figure 2D** montre une variante dans laquelle le canal présente un élargissement tridimensionnel, de forme pyramidale.

La **figure 2E** montre une variante de canal proche de celui de la **figure 2B****.**

La **figure 2F** montre une variante de canal proche de celui de la **figure 2A****,** mais avec une forme de losange essentiellement symétrique.

La **figure 2G** montre une variante avec une entrée 4 ramifiée en trois canaux d'alimentation, en amont du canal.

La **figure 2H** montre une variante avec quatre sorties 5, débouchant sur des canaux avals respectifs parallèles. Dans cette variante, la largeur du canal croît continûment de l'entrée 4 jusqu'aux sorties 5, sans passer par un maximum, et la forme du canal vu de dessus est donc globalement triangulaire.

La **figure 2I** montre une variante proche de celle de la **figure 2H**, avec seulement deux sorties 5.

La **figure 2J** montre une variante avec de multiples ramifications en aval de l'entrée 4, de type delta.

La **figure 2K** montre une variante avec trois sorties 5, débouchant sur des canaux avals dans des directions divergentes.

La **figure 2L** montre une variante dans laquelle le canal est ramifié en trois branches entre l'entrée 4 et la sortie 5, disposées parallèlement dans le même plan du substrat du système microfluidique, chaque branche comportant une zone de capture 3 (avec élargissement de la branche de l'entrée 4 vers la sortie 5).

La **figure 2M** montre une variante dans laquelle le canal est ramifié en trois branches entre l'entrée 4 et la sortie 5, superposées en différentes épaisseurs du substrat du système microfluidique, chaque branche comportant une zone de capture 3 (avec élargissement de la branche de l'entrée 4 vers la sortie 5). Cette disposition permet notamment d'augmenter le débit global du système à partir d'une forme unique, en conservant ses caractéristiques.

La **figure 2N** montre une variante avec un canal formant un coude au niveau de la sortie 5.

La **figure 2O** montre une variante avec un canal formant un coude au niveau de l'entrée 4.

La **figure 2P** montre une variante avec un canal comportant deux entrées 4, le canal comportant deux branches qui se rejoignent, et une sortie 5 unique en aval du point de jonction.

### Application du champ magnétique

En faisant à nouveau référence à la **figure 1**, l'invention prévoit des moyens d'application d'un champ magnétique 6, le champ magnétique appliqué étant essentiellement parallèle à l'axe longitudinal 7 du canal 2. Sur la figure, l'orientation du champ magnétique est illustrée par une flèche.

En particulier, la direction du champ magnétique en tout point de la zone de capture 3 du canal 2 forme un angle inférieur ou égal à 20°, ou à 15°, ou à 10°, ou à 5°, par rapport à l'axe longitudinal 7 du canal 2 ; ou encore la direction du champ magnétique en tout point de la zone de capture 3 du canal est parallèle à l'axe longitudinal 7 du canal.

Alternativement, on peut prendre en considération le vecteur moyen du champ magnétique sur une section transversale du canal. Ce vecteur moyen forme un angle inférieur ou égal à 20°, ou à 15°, ou à 10°, ou à 5° avec l'axe longitudinal du canal, le long de la zone de capture du canal ; ou encore ce vecteur moyen est parallèle à l'axe longitudinal du canal le long de la zone de capture.

Cet alignement du champ magnétique sur la géométrie du canal se traduit aussi en termes d'alignement du champ magnétique avec l'écoulement du fluide.

Ainsi, selon des modes de réalisation préférés :
- la direction du champ magnétique en tout point de la zone de capture du canal forme un angle inférieur ou égal à 20°, ou à 15°, ou à 10°, ou à 5°, par rapport au vecteur vitesse du fluide dans le canal en ce point (en écoulement non-turbulent) ;
- le vecteur moyen du champ magnétique sur une section transversale du canal forme un angle inférieur ou égal à 20°, ou à 15°, ou à 10°, ou à 5° par rapport à, ou encore est parallèle avec, le vecteur vitesse moyen du fluide sur ladite section transversale, le long de la zone de capture du canal.

Une autre manière essentiellement équivalente de définir le parallélisme du champ magnétique par rapport à la zone de capture du canal (à quelques divergences ou faibles variations locales ou temporelles près) consiste à imposer que les lignes de champ magnétique dans la zone de capture du canal soient essentiellement alignées avec les lignes de champ hydrodynamique dans cette zone de capture (en écoulement non-turbulent), c'est-à-dire que l'angle entre ces lignes de champ respectives est inférieur ou égal à 20°, ou à 15°, ou à 10°, ou à 5° en tout point de la zone de capture.

La composante du champ magnétique dans le plan du substrat perpendiculairement à l'axe longitudinal 7 du canal 2 (composante latérale) est en tout point inférieure à 30 %, ou à 20% (ou à 15 %, ou à 10 %, ou à 5 %) de la composante du champ selon l'axe longitudinal, et le champ est donc orienté quasiment selon l'axe longitudinal 7 dans l'ensemble du canal 2. Il est à noter que la présence d'une décroissance du champ le long de l'axe longitudinal implique, par conservation du flux, une divergence du champ, et qu'en certains points, et notamment vers les parois du canal, le champ présente par conséquent une composante latérale non nulle.

Ce champ magnétique peut être créé au moyen d'un aimant permanent, d'un électroaimant, ou d'une combinaison de ceux-ci, éventuellement en association avec une pièce polaire formée avec un matériau magnétique doux susceptible de diriger les lignes de champ. De préférence, une telle pièce polaire est dépourvue de toute microstructure susceptible de créer une pluralité de maxima locaux du champ magnétique.

Dans le cas de l'utilisation d'un électroaimant sans noyau, par exemple une bobine électrique, on considère que les pôles de l'électroaimant sont les deux plans correspondant à l'entrée et à la sortie du flux magnétique dans la bobine.

Dans la suite, sauf mention contraire, on désignera les aimants permanents et les électro-aimants sous le terme générique « aimant ».

Dans certains modes de réalisation, le champ magnétique peut être activable ou réglable. En particulier, il est avantageux de pouvoir modifier l'intensité (l'amplitude) du champ magnétique, sans modifier son orientation (c'est-à-dire sa direction, ou la forme des lignes de champ).

L'invention prévoit de disposer l'aimant avec son axe polaire nord / sud essentiellement aligné avec l'axe longitudinal du canal. Il est préféré de disposer l'aimant du côté de l'entrée du canal. Cela diffère de géométries proposées dans l'état de la technique, où les aimants ou électroaimants sont disposés sur les côtés du canal ou au-dessus et / ou en-dessous du canal, c'est-à-dire en tout état de cause près des parois du canal essentiellement parallèles à la direction de l'écoulement.

La face de la partie polaire ou de l'aimant permanent faisant face à la chambre de capture peut, selon les modes de mise en oeuvre, être plate, ou présenter diverses formes. Par exemple, elle peut être courbée selon une ou plusieurs directions, ou même dans certain cas comporter une ou plusieurs arêtes. Elle est de préférence configurée de façon à générer un champ qui décroit de façon progressive et continue à l'intérieur de la zone de capture. S'il existe des arêtes, elles doivent donc de préférence présenter un angle obtus, préférablement supérieur à 60°, ou supérieur à 80°. De même, si ladite face de la partie polaire ou de l'aimant est courbe et convexe (bombée), elle présente de préférence un rayon de courbure supérieur à la longueur de la portion de la zone de capture destinée à recevoir et à retenir des particules magnétiques. L'ajustement de la forme de la pièce polaire (ou de l'aimant) peut permettre de moduler la divergence du champ magnétique et donc d'optimiser les performances du système, en fonction de la forme du canal et de sa zone de capture, soit par essais et erreurs, soit par simulation numérique.

Ainsi, le champ magnétique prévu dans l'invention varie de préférence de manière continue et monotone (décroissante) le long de l'axe longitudinal ou de la direction d'écoulement, de l'entrée vers la sortie, dans la zone de capture. Tout maximum local d'intensité est ainsi évité, qui pourrait conduire à un piégeage local et donc compact de particules magnétiques.

Des variations non linéaires, voire non continues, de la section transversale du canal, de la vitesse moyenne d'écoulement du fluide, et de l'intensité du champ magnétique, sont possibles dans certains cas, selon les débits et temps de résidence du fluide souhaités.

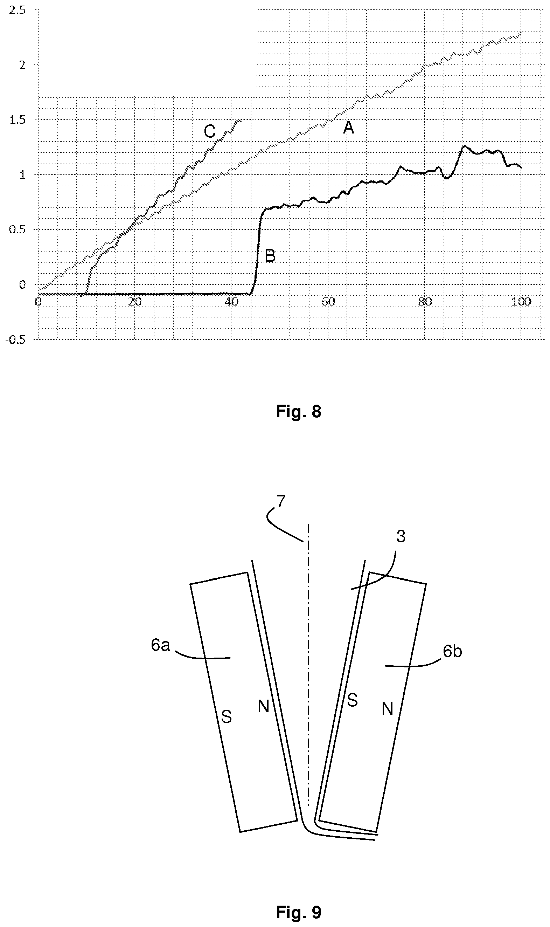

Selon un mode de réalisation alternatif, illustré à la **figure 9**, le champ magnétique est essentiellement orthogonal à l'axe longitudinal 7 du canal, au lieu d'être essentiellement parallèle. Par exemple, les moyens d'application d'un champ magnétique 6 peuvent comprendre un premier aimant 6a et un deuxième aimant 6b, situés de part et d'autre de la zone de capture 3 du canal et se faisant face, le pôle nord du premier aimant 6a étant dirigé vers le canal, et le pôle sud du deuxième aimant 6b étant dirigé vers le canal.

La face du pôle nord du premier aimant 6a n'est pas parallèle à la face du pôle sud du deuxième 6b, de sorte que l'intensité du champ magnétique soit décroissante dans la zone de capture 3 selon la direction d'écoulement (les faces des aimants sont plus proches du côté amont que du côté aval dans la zone de capture 3).

Dans l'exemple illustré, la zone de capture 3 est délimitée par des côtés du canal non parallèles, et qui s'écartant dans le sens de l'écoulement, et les aimants 6a et 6b sont disposés parallèlement à ces côtés du canal.

### Lit de particules magnétiques

L'arrangement décrit ci-dessus permet de former un lit de particules magnétiques dans la zone de capture du canal. Plus précisément, on injecte dans le canal un fluide contenant des particules magnétiques, et on applique le champ magnétique simultanément (ou après le début de l'injection du fluide contenant les particules magnétiques).

En réglant correctement l'intensité du champ magnétique et le débit de fluide, on réalise un équilibre entre les forces magnétiques et hydrodynamiques permettant de maintenir les particules magnétiques dans la zone de capture. Il est ensuite possible d'introduire d'autres fluides dans le canal, dépourvus de particules magnétiques, et de les faire passer à travers le lit de particules magnétiques retenu dans la zone de capture.

De préférence, le lit de particules magnétiques dans le système est un lit dense mais non compact. Ainsi, les particules se déplacent les unes relativement aux autres.

Un état compact est défini comme un état dans lequel les particules sont en contact permanent ou quasi-permanent avec les particules voisines. Un état dense mais non compact est un état dans lequel les particules entrent en contact les unes avec les autres occasionnellement, mais dans lequel les particules sont de manière prépondérante en contact avec le fluide, et non avec d'autres particules (la distance moyenne entre particules étant toutefois de préférence inférieure à 100 fois, ou à 50 fois, ou à 20 fois, ou à 15 fois, ou à 10 fois, ou à 5 fois, ou à 3 fois la taille moyenne des particules).

Ainsi, l'état dense mais non compact qui est recherché dans le cadre de l'invention correspond généralement, pour des particules sphériques ou approximativement sphériques, à une fraction volumique occupée par les particules de 0,01 à 0,3 dans la zone de capture, par exemple de 0,01 à 0,05 ou alternativement de 0,05 à 0,3. A titre de comparaison, un empilement compact de particules sphériques ou approximativement sphériques correspond généralement à une fraction volumique occupée par les particules de 0,4 à 0,6.

Pour des particules non sphériques, et notamment pour des particules très allongées, l'état dense mais non compact qui est recherché dans le cadre de l'invention peut correspondre à une fraction volumique plus faible, par exemple de 0,001 à 0,1, puisque ces particules ont tendance à interagir plus facilement les unes avec les autres, et ont plus de mal à atteindre un état compact.

On peut également faire référence à la porosité du lit de particules magnétiques, qui est définie comme étant la fraction volumique non occupée par les particules magnétiques. Cette porosité du lit de particules magnétiques vaut par exemple de 50 à 95 %, ou de préférence de 60% à 90%.

Dans certains modes de réalisation des procédés de l'invention, dans certaines étapes la zone de capture comprend un lit dense mais non compact de particules magnétiques, comme décrit ci-dessus, et dans d'autres étapes elle comprend un lit compact de particules magnétiques, comme décrit ci-dessus (avec par exemple une fraction volumique occupée de 0,4 à 0,6), le passage d'un régime à l'autre pouvant être effectué par ajustement du débit, de la pression ou du champ magnétique.

### Moyens supplémentaires associés au canal du système microfluidique

Le système selon l'invention comporte un ensemble de moyens permettant d'assurer l'injection et la circulation contrôlée de fluides dans le canal.

Ainsi, ce système peut comporter une pluralité d'autres canaux, d'entrées et sorties supplémentaires et de vannes, ainsi que des réservoirs des différents fluides utilisés.

Dans l'exemple illustré à la **figure 1**, le système microfluidique 1 comporte un canal d'alimentation 8 connecté fluidiquement à l'entrée 4 du canal 2 décrit ci-dessus. Ce canal d'alimentation 8 comporte une première entrée 9, une deuxième entrée 10 et une troisième entrée 11, chaque de ces entrées pouvant être connectée à un réservoir d'un fluide distinct et / ou à des moyens de contrôle de la pression. En sortie 5 du canal 2 décrit ci-dessus est connectée une conduite secondaire 13, elle-même connectée à une sortie 12 qui peut être connectée à un réservoir de fluide et / ou à des moyens de contrôle de la pression.

Dans l'exemple illustré, le canal d'alimentation 8 forme un coude d'un angle supérieur à 90° avec le canal 2 principal, ce qui peut être avantageux pour amener des particules magnétiques depuis un réservoir jusqu'au canal 2.

Le caractère régulier de la courbure, combiné à la faible section du canal dans cette zone dans lequel le champ magnétique n'est pas dirigé selon l'axe du canal, et qui ne présente donc pas le caractère d'une zone de capture selon l'invention, permet d'éviter que certaines particules magnétiques restent piégées dans cette section du canal, et permet au contraire de contraindre la plus grande partie, et dans les cas les plus favorables la totalité, des particules magnétiques contenues dans le canal, à rejoindre la zone de capture 3 et à y rester. Mais toute autre conformation, notamment dans l'alignement du canal 2, est possible. De même, dans l'exemple illustré, la conduite secondaire 13 est alignée selon l'axe longitudinal 7 du canal 2, mais toute autre conformation est possible.

Le système selon l'invention comporte avantageusement (ou est connecté, généralement par des conduites de fluide, à) des moyens de détection, tels que des moyens de détection optique (par exemple de détection par luminescence, fluorescence, phosphorescence, absorption lumineuse, diffraction, réfractométrie, résonance plasmon) ou des moyens de détection électrique (par exemple de détection par impédancemétrie, conductimétrie, électrochimie, voltamétrie cyclique), ou encore des moyens de détection acoustique, par exemple des capteurs à base de matériaux piézoeletriques tes que des microbalances à quartz ou des résonateurs à ondes de surface.

Le système peut comporter ou être connecté à des dispositifs d'analyse, des connecteurs ou des réacteurs chimiques ; par exemple à un spectromètre de masse, à un dispositif d'amplification d'acide nucléique, à une « puce à ADN » ou « puce à protéine » souvent appelé « microarray », un séquenceur d'acide nucléique, à un dispositif d'électrophorèse, à un filtre, à un mélangeur ou autre.

Le système selon l'invention comporte avantageusement des moyens de déplacement de fluide, susceptibles de déplacer une pluralité de fluides tels que des réactifs, échantillons, solutions de rinçage, solutions colloïdales, de manière contrôlée dans le canal, et également en provenance des réservoirs, ou vers d'éventuels instruments de collecte ou de détection. Ces moyens de déplacement de fluide peuvent comprendre des pompes microfabriquées ou des pompes externes, telles que des pompes de contrôle microfluidique, des pompes de seringue, des pompes péristaltiques, des pompes à membrane, des pompes à piston, des pompes rotatives.

De préférence, on utilise des pompes sans impulsion, telles que des pompes microfluidiques contrôlées par pression. De tels systèmes évitent les problèmes d'hystérésis qui peuvent se rencontrer avec les systèmes de pompage fondés sur un contrôle du volume, tels que les pompes à seringue. En effet dans ces derniers, chaque arrêt du flux a tendance à engendrer un empilement compact de particules magnétiques vers l'entrée du canal, bouchant l'entrée du canal et pouvant conduire à une accumulation de pression, puis à un éclatement de l'empilement compact de particules et à la perte de particules lorsque le débit est rétabli.

Les pompes microfluidiques contrôlées par pression, comme par exemple le système MFCS de Fluigent, ou la pompe Mythos de Dolomite, permettent d'éviter d'accumuler une pression excessive, et de limiter les risques ci-dessus.

Il est également préféré de recourir à des systèmes dans lesquels la pression est régulée de manière dynamique grâce aux informations provenant d'un débitmètre. Cela peut permettre d'augmenter progressivement la pression afin d'augmenter ou diminuer rapidement le débit de fluide à travers le lit de particules magnétiques afin de maintenir le débit dans des limites prédéterminées et ainsi d'éviter tout phénomène d'éclatement du lit de particules.

En effet, avec ce mode de réalisation, on peut arrêter le flux, ou réduire le débit à une faible valeur, tout en conservant le champ magnétique, et appliquer une pression positive finie et contrôlée à l'entrée du canal. Ainsi, on évite le retour en arrière des particules magnétiques dans la zone du canal située en amont de la zone de capture.

En général les valeurs de débit avec lesquelles les particules magnétiques sont dans un état compact sont inférieures de 5 à 50 fois, voire jusqu'à 500 fois par rapport aux valeurs de débit avec lesquelles le lit de particules magnétiques dans un état dense mais non compact.

Le système microfluidique de l'invention peut aussi être associé à, ou peut comprendre, tout contrôleur informatique, électronique ou électrique, afin par exemple de contrôler la température et le fonctionnement des différents composants, d'automatiser les opérations et d'enregistrer des données.

Dans le cadre des procédés pouvant être mis en oeuvre selon l'invention, on fait circuler des fluides dans le système microfluidique, et notamment dans le canal décrit ci-dessus. Les procédés peuvent comprendre des périodes de circulation continue de fluide, en alternance avec des périodes d'arrêt de fluide (pouvant notamment permettre des étapes d'incubation ou de réaction). Ils peuvent comprendre des périodes de circulation de fluide à un premier débit, en alternance avec des périodes de circulation de fluide à un deuxième débit qui est de préférence au moins 10 fois, ou au moins 50 fois inférieur au premier débit.

Des valeurs de débit pouvant être utilisées sont notamment de 1 nL/min à 10 mL/min, et en particulier de 1 µL/min à 100 µL/min.

Il est particulièrement intéressant de mettre en oeuvre l'invention dans un mode d'injection de fluide en entrée du système microfluidique et de collecte de fluide en sortie du système.

### Conduite secondaire de résistance hydraulique élevée

Selon un mode de réalisation, le système selon l'invention comporte une conduite secondaire présentant une résistance hydraulique supérieure à celle du canal précité, soit en amont (avant l'entrée du canal) soit en aval (après la sortie du canal), en série par rapport au canal (principal) décrit ci-dessus. Ce mode de réalisation permet un meilleur contrôle du flux dans le canal.

Dans l'exemple de la **figure 1**, la conduite secondaire 13 présente une résistance hydraulique supérieure à celle du canal 2, par un choix judicieux de sa longueur et de sa section transversale (qui peut être par exemple égale, ou inférieure ou égale, à la section transversale du canal 2 au niveau de sa sortie 5).

De préférence la résistance hydraulique de la conduite secondaire est au moins 2 fois, ou au moins 5 fois, ou au moins 10 fois, par exemple entre 10 fois et 100 fois celle de la zone de capture du canal décrit ci-dessus (en l'absence de particules magnétiques).

La conduite secondaire peut être un canal secondaire formé dans le même substrat que le canal, ou il peut s'agir d'un tube ou tuyau connecté au canal.

### Dispositif oscillant

Selon un mode de réalisation, le système selon l'invention comporte, outre des moyens de déplacement du fluide de l'entrée vers la sortie, un dispositif oscillant, c'est-à-dire un dispositif adapté à générer des oscillations de fluide dans le canal (et notamment dans tout ou partie de la zone de capture). Le dispositif oscillant peut générer des vibrations mécaniques ou des oscillations pneumatiques. Il est de préférence choisi parmi les transducteurs soniques, ultrasoniques, ou piézoélectriques, les éléments vibrants (notamment les moteurs rotatifs ou alternatifs reliés à une masse), les haut-parleurs et les pistons oscillants. Le dispositif oscillant peut permettre de provoquer des inversions temporaires du flux.

Les oscillations ainsi générées peuvent avoir par exemple une fréquence de 1 Hz à 10 MHz, par exemple de 2 Hz à 2 kHz, notamment de 2 à 20 Hz, ou de 20 Hz à 200 Hz, ou de 200 Hz à 2 kHz. Une gamme de 10 Hz à 1 kHz est préférée.

Le dispositif oscillant permet d'obtenir une circulation de particules magnétiques plus homogène, et d'augmenter l'efficacité du transport des analytes vers les particules magnétiques.

Dans le cas où le dispositif oscillant génère des vibrations mécaniques, il peut être placé en communication sonique ou mécanique directe avec le canal, soit en le fixant directement au canal ou à son support (éventuellement par l'intermédiaire d'une tige ou autre structure conduisant les vibrations sonores) ; alternativement, il peut être fixé sur un tube ou tuyau flexible en communication fluidique avec le canal (notamment tube d'alimentation ou de collecte de fluide).

Comme montré dans les exemples ci-dessous, un dispositif oscillant permet dans certains cas, et en particulier quand les particules ont tendance à s'agréger, à cause de leur nature, ou à cause du fluide dans lequel elles sont contenues, d'améliorer la stabilité de l'écoulement et l'homogénéité du lit de particules magnétiques. Il permet aussi dans certains cas d'augmenter le débit, et d'abaisser la pression seuil de fonctionnement.

### Fluides traités selon l'invention

Certains fluides utilisés dans le cadre de l'invention sont de préférence des suspensions colloïdales, c'est-à-dire des fluides contenant des objets colloïdaux.

Par objets colloïdaux, on entend des composés organiques ou inorganiques, naturels ou artificiels, tels que des cellules, organelles, virus, agrégats de cellules, îlots de cellules, embryons, grains de pollen, particules organiques naturelles ou artificielles (par exemple en latex de polymère), dendrimères, vésicules, particules magnétiques, quantum dots, particules métalliques, particules organométalliques, particules d'oxydes métalliques, particules de céramique, particules de silice, particules de verre, liquides organiques, hydrogels, nanotubes, macromolécules naturelles ou artificielles, microgels, agrégats macromoléculaires, protéines ou agrégats de protéines, polynucléotides ou agrégats de polynucléotides, agrégats nucléoprotéiques, polysaccharides, assemblages supramoléculaires ou combinaison de ceux-ci.

Le procédé de l'invention est notamment mis en oeuvre en utilisant au moins une suspension colloïdale de particules (microparticules ou nanoparticules) magnétiques. Ces particules peuvent avoir diverses tailles, formes et compositions. Elles peuvent aussi être présentes dans diverses distributions de taille, forme et composition. La taille des particules est généralement la dimension moyenne massique des particules. Pour des particules approximativement sphériques, cette dimension correspond au diamètre, et pour des particules non-sphériques, elle correspond à la dimension maximale.

De préférence, tout ou partie des particules magnétiques sont des particules super-paramagnétiques. De telles particules ne conservent pas de moment magnétique permanent en l'absence de champ magnétique externe, et elles peuvent donc se déplacer plus facilement les unes par rapport aux autres, être conservées dans un état dispersé, et être retirées de la zone de capture en modifiant le champ magnétique dans cette zone.

Selon des modes de réalisation, on utilise des particules magnétiques ayant une distribution monomodale avec une taille allant de 10 nm à 100 µm, de préférence de 100 nm à 10 µm.

Selon d'autres modes de réalisation, on utilise des particules magnétiques ayant une distribution de taille bimodale ou multimodale, avec une première catégorie de particules ayant une taille (moyenne) allant de 500 nm à 10 µm, et une deuxième catégorie de particules ayant une taille (moyenne) allant de 10 à 100 nm. On peut aussi utiliser des particules magnétiques ayant une distribution de taille bimodale, avec une première catégorie et une deuxième catégorie de particules, la taille (moyenne) de la deuxième catégorie étant de 5 à 500 fois inférieure à la taille (moyenne) de la première catégorie.

Des particules magnétiques susceptibles d'être utilisées dans le cadre de l'invention sont notamment celles proposées par les sociétés Dynal, Miltenyi, Estapor, polysciences, Ademtech, et autres. On peut également utiliser des particules magnétiques synthétisées *ad hoc.*

L'invention permet également de manipuler une combinaison de particules magnétiques et non-magnétiques.

Dans le cadre de l'invention, on manipule également des échantillons de fluide, c'est-à-dire des fluides contenant des analytes ou des substrats d'intérêt. Il peut s'agir de fluides corporels, de fluides extraits d'un échantillon solide ou liquide dans lequel les analytes sont initialement présents, ou d'un fluide artificiel tel qu'un tampon, dans lequel les analytes ont été dissouts ou suspendus.

### Analytes, substrats et ligands traités ou utilisés selon l'invention

Les analytes ou substrats peuvent être toute espèce chimique ou biologique, à condition qu'elle puisse être suspendue ou dissoute dans un fluide. Les analytes ou substrats peuvent être des molécules, des ions, des atomes, des macromolécules ou des objets colloïdaux (tels que définis ci-dessus). Il peut y avoir un type unique d'analyte ou substrat ou une pluralité de types d'analytes dans un échantillon de fluide.

En général, les analytes ou substrats visés dans le cadre de la présente demande sont des espèces que l'on souhaite séparer d'un échantillon de fluide, ou concentrer au sein de ce fluide, ou échanger d'un premier fluide vers un second fluide, pour les examiner, les analyser, les stocker, les identifier, les cultiver, les utiliser pour une synthèse et / ou les modifier chimiquement, biologiquement ou physiquement.

Généralement, on utilise le mot « analyte » pour des espèces qu'on souhaite analyser sans les modifier, et « substrat », pour des espèces qu'on souhaite modifier, mais dans certaines applications des espèces peuvent jouer tour à tour le rôle de substrat et d'analyte, par exemple si on doit dans un premier temps les modifier pour pouvoir les détecter. Aussi, dans la description, les deux mots « analyte » et « substrat » pourront être substitués l'un à l'autre en restant dans le cadre de l'invention.

De préférence, les particules magnétiques utilisées dans le cadre de l'invention (susceptibles d'être piégées dans la zone de capture du canal) portent des ligands à leur surface.

Alternativement, ou en outre, peut prévoir que des ligands soient portés par d'autres objets colloïdaux, eux-mêmes susceptibles de se lier aux particules magnétiques utilisées dans le cadre de l'invention (susceptibles d'être piégées dans la zone de capture du canal).

Le terme de ligand désigne une espèce ou une fonction susceptible de se lier de façon réversible ou irréversible à une autre espèce, en l'occurrence un analyte.

Les ligands de l'invention peuvent être toute espèce chimique, physique ou biologique susceptible d'être fixée à la surface des particules magnétiques (et éventuellement d'autres objets colloïdaux).

Les analytes et les ligands de l'invention peuvent être par exemple des acides nucléiques, des polypeptides, des acides aminés, des composés chimiques (ou des fonctions chimiques en ce qui concerne les ligands), des enzymes, des catalyseurs.

Le terme acide nucléique désigne les acides nucléiques naturels (par exemple ADN et ARN), mais aussi les acides nucléiques modifiés ou artificiels tels que acides nucléiques bloqués, acides nucléiques peptidiques, acides nucléiques thiolés, et autres. Il comprend les acides nucléiques génomiques, ribosomiques, mitochondriaux, les acides nucléiques d'organismes pathogènes, l'ARN messager, le micro-ARN, et les acides nucléiques médicaments.

Le terme polypeptide est pris dans son sens général et désigne toute molécule ou tout assemblage moléculaire comprenant au moins une séquence d'acides aminés, notamment les protéines naturelles et artificielles, les fragments de protéines, les complexes protéiques, les enzymes, les anticorps, les glycopeptides, les glycoprotéines et les modifications chimiques et biochimiques de ceux-ci.

Des ligands d'intérêt particulier pour la mise en oeuvre de l'invention sont les anticorps, les métaux, l'histidine, les groupements hydrophobes, les groupements de liaison hydrogène, la protéine A, les séquences d'acide nucléique chargées, les polyélectrolytes, les phospholipides, les composés chimiques, les médicaments, les groupements fluorescents, les groupements luminescents, les colorants, les nanoparticules (notamment d'or), les quantum dots, les agents intercalants de l'ADN, les aptamères, les mélanges utilisés pour l'amplification de l'ADN, les espèces susceptibles d'affecter le métabolisme des cellules ou les propriétés (notamment les propriétés optiques) d'objets colloïdaux.

La liaison des ligands aux analytes peut être irréversible, ce qui signifie que cette liaison ne peut être rompue sans détruire ou altérer de manière importante l'intégrité de l'analyte et / ou de la particule magnétique (ou autre objet colloïdal). Par exemple, il peut s'agir d'une liaison covalente, ou encore de la liaison de protéines dénaturées sur une surface, de l'attachement irréversible de latex sur une surface par séchage ou application de chaleur, ...

La liaison des ligands aux analytes peut aussi être réversible, c'est-à-dire qu'elle peut être rompue sans modifier de manière significative les espèces liées. Il peut s'agir notamment d'une liaison de type physique, d'une liaison par interaction hydrophobe ou électrostatique ou diélectrostatique ou par interaction hydrogène ou par réaction chimique réversible.

Une liaison par interaction chimique, par exemple par hybridation de brins d'acide nucléique, par interaction antigène-anticorps, par interaction aptamère-protéine, peut être réversible ou irréversible.

Comme exposé ci-dessus, l'invention permet de retenir dans la zone de capture du canal un lit de particules magnétiques, par application du champ magnétique (et ce pendant une durée supérieure au temps de résidence des particules dans la zone de capture en l'absence de champ magnétique). On peut aussi prévoir plusieurs zones de capture le long d'un canal ou de plusieurs canaux dans le système microfluidique. Un des avantages de l'invention est que, bien qu'étant retenues dans la ou les zones de capture, les particules magnétiques sont toutefois susceptibles de circuler à l'intérieur de cette ou ces zones de capture.

Ce lit de particules magnétiques est susceptible à son tour de retenir des analytes circulant dans le canal, soit par liaison directe des analytes aux particules magnétiques, soit par liaison indirecte des analytes aux particules magnétiques, *via* d'autres objets colloïdaux ; ou est susceptible de réagir ou interagir d'une quelconque façon avec ces analytes (directement ou indirectement, *via* d'autres objets colloïdaux).

On peut également prévoir une ou des étapes d'élution des analytes retenus dans la zone de capture, par injection d'un fluide d'élution approprié.

On peut également utiliser un deuxième ligand, ou ligand supplémentaire, qui peut être injecté après une étape de liaison (directe ou indirecte) des analytes aux particules magnétiques dans la zone de capture, et qui peut avantageusement faciliter la détection de la liaison des analytes aux particules magnétiques. Il peut s'agir par exemple d'un anticorps secondaire, lié éventuellement à un fluorophore, ou lié éventuellement à une enzyme. Dans ce cas, la détection de la liaison ligand / analyte / ligand supplémentaire peut être effectuée en ajoutant un substrat pour l'enzyme en question, par exemple un substrat fluorogénique ou chimioluminescent.

### Applications de l'invention

L'invention permet de mettre en oeuvre dans le système microfluidique décrit ci-dessus des réactions ou séparations chimiques, biochimiques ou biologiques, telles que notamment des réactions catalytiques, des hybridations, des réactions électrochimiques, des réactions enzymatiques, des immunodosages, des séparations chromatographiques, des réactions de chimioluminescence, des captures immunologiques, des captures par affinité, des élutions, des purifications, des concentrations, des extractions et des combinaisons de celles-ci.

Selon certains modes de réalisation, l'invention est mise en oeuvre dans le cadre de procédés d'immunodosage, ou de test génétique ou de test ELISA.

Selon certains modes de réalisation, l'invention est mise en oeuvre dans le cadre d'une analyse comprenant une amplification d'acide nucléique, par exemple une amplification isotherme par PCR, telle que décrite par exemple dans A. Niemz et al., Trends in Biotechnology, mai 2011, vol. 29, n°5, p.240-250.

Selon certains modes de réalisation, l'invention est mise en oeuvre pour effectuer un traitement chimique d'espèces contenues dans un fluide qui est injecté dans la zone de capture en présence de particules magnétiques.

Le système microfluidique selon l'invention peut être associé à, ou peut comprendre, tout instrument permettant la mise en oeuvre des modes de réalisation ci-dessus, notamment tout instrument d'analyse, de contrôle, de production, le cas échéant sous forme d'un système d'analyse miniaturisé ou d'un microréacteur.

L'invention peut être mise en oeuvre dans le cadre de dispositifs et procédés de recherche, de diagnostic, d'analyse, de synthèse, de contrôle de qualité, et ce en médecine, en biologie, en sciences de la vie, dans l'agroalimentaire, l'industrie cosmétique, la pharmacie, l'analyse légale, la sécurité, la biosécurité, l'industrie énergétique (notamment pour la manipulation de matériaux radioactifs) ou la chimie.

L'invention peut permettre de capturer, trier, extraire, purifier, analyser, identifier ou cultiver des analytes issus d'un échantillon de fluide.

L'invention peut permettre de modifier des analytes contenus dans un fluide, par exemple par réaction catalytique, chimique, biochimique ou enzymatique.

L'invention peut notamment permettre de capturer des cellules ou des virus dans un milieu, de les identifier ou de les lyser. Cela peut notamment être intégré à des dispositifs et procédés de diagnostic, notamment de diagnostic de maladies infectieuses, de cancers, de maladies cardiovasculaires, ou de diagnostic prénatal. Cela peut également être intégré à des dispositifs et procédés de contrôle qualité, de maîtrise des contaminations, notamment bactériennes, virales ou chimiques, dans les domaines agro-alimentaire, de l'énergie notamment nucléaire, de la qualité de l'eau, de la chimie, de l'environnement, de la sécurité notamment de la bio-sécurité.

Les cellules en question peuvent être des bactéries, champignons, cellules eucaryotes, notamment des cellules tumorales circulantes, des cellules hématopoïétiques, des globules rouges, des cellules endothéliales circulantes, des parasites, des cellules foetales circulantes.

### EXEMPLES

Les exemples suivants illustrent l'invention sans la limiter.

### Exemple 1 - champ hydrodynamique et champ magnétique dans un système selon l'invention

On réalise un système microfluidique conformément à la **figure 1**. Le canal 2 fait 21 mm de longueur et 50 µm d'épaisseur, avec une largeur variant de 100 µm à 2 mm (zone de largeur maximale).

Le système microfluidique est préparé en polydiméthylsiloxane selon le protocole de microlithographie décrit dans l'article de Mohamadi et al. dans Biomicrofluidics, 5,044114 (2011);

Pour la génération du champ magnétique, on utilise un aimant permanent de type NdFeB1, placé à proximité du canal 2 : la distance entre l'aimant et le système microfluidique est de 2 mm. L'aimant est magnétisé dans sa plus grande dimension et présente un champ magnétique rémanent de 1,47 T. Il présente une taille de 30 x 20 x 20 mm

Des simulations ont été effectuées avec 3D COMSOL afin d'évaluer l'intensité du champ magnétique dans le canal 2 et les forces exercées sur les particules magnétiques.

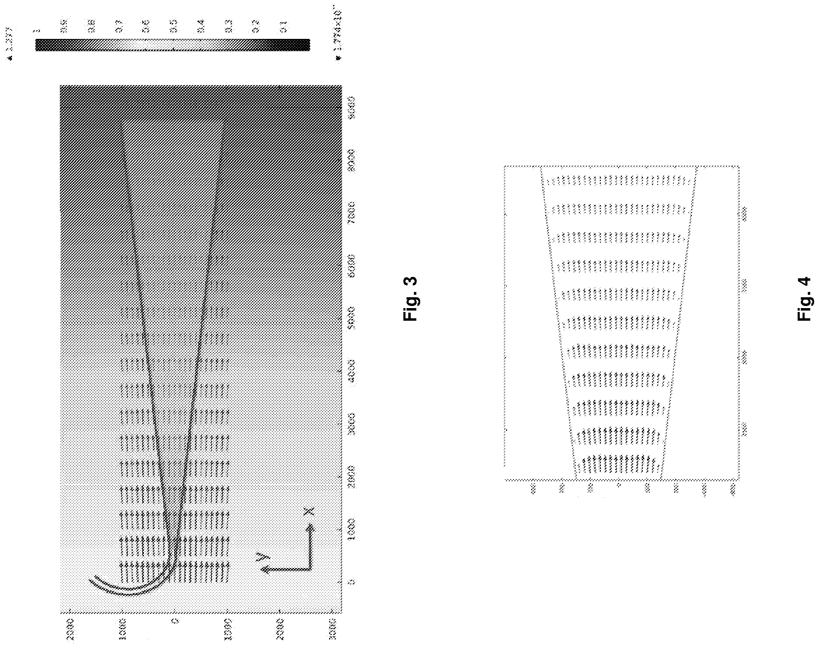

Les résultats sont représentés sur la **figure 3**. On constate que l'intensité maximale du champ magnétique est d'environ 0,7 T au niveau de l'entrée 4 du canal 2. Elle décroit jusqu'à 0,36 T à 1 mm de distance.

La composante du champ magnétique dans le plan du substrat perpendiculairement à l'axe longitudinal 7 du canal 2 (composante latérale) est petite par rapport à la composante du champ selon l'axe longitudinal, et le champ est donc orienté quasiment selon l'axe longitudinal 7 dans l'ensemble du canal 2.

Connaissant l'intensité du champ en tout point il est possible de calculer la force magnétique exercée sur une particule magnétique, à partir de la polarisabilité magnétique de cette particule. Dans cet exemple on a utilisé des particules de marque DYNAL® à 2,8 µm.

De même, en utilisant la libraire hydrodynamique COMSOL, on a calculé en tout point la vitesse d'écoulement pour un débit donné, en régime laminaire (bas nombre de Reynolds). La **figure 5** fournit un exemple de champ hydrodynamique calculé pour un débit de 3 µL/min.

La vitesse du fluide diminue le long de l'axe longitudinal, de 4,6 mm/s à 0,5 mm/s en 3 mm. Le profil de vitesse est quasi plat dans la direction transverse, la vitesse ne tendant vers zéro qu'à proximité immédiate des parois. En d'autres termes, la force de frottement exercée sur les particules magnétiques est quasi-uniforme sur la largeur du canal (ce qui est confirmé par observation expérimentale).

A partir du profil de vitesse et du profil du champ magnétique, on peut calculer les forces magnétique et hydrodynamique exercées en tout point du canal, pour un écoulement donné et pour une particule isolée.

Les résultats sont représentés sur la **figure 5**. La courbe A représente la force magnétique, la courbe B représente la force hydrodynamique pour un débit de 6 µL/min, et la courbe C représente la force hydrodynamique pour un débit de 3 µL/min.

On en déduit que, à un débit de 6 µL/min, les particules magnétiques sont entraînées par le flux et ne sont pas piégées dans le canal (du fait que la force hydrodynamique est supérieure à la force magnétique en tout point du canal), mais que, à un débit de 3 µL/min, il existe une zone d'équilibre stable, c'est-à-dire une zone de capture permettant de retenir les particules magnétiques.

Ainsi, par des simulations numériques relativement simples, on peut calculer pour toute forme de canal et toute forme d'aimant ou de pièce polaire les champs de force hydrodynamique et magnétique, et ainsi optimiser les paramètres du système pour un type de particules données et une application donnée (par exemple si un débit particulier est souhaité).

### Exemple 2 - mise en évidence d'un lit fluidisé de particules magnétiques

On utilise le système de la **figure 1**, ainsi que, à titre de comparaison, le même système sans la conduite secondaire 13. Le système est relié à un dispositif de contrôle des écoulements de type MAESFLO (Fluigent).

La **figure 6A** illustre le comportement du système (débit en fonction de la pression) sans la conduite secondaire 13. La courbe A est obtenue en l'absence de particules magnétiques. Le canal 2 se comporte comme une résistance hydraulique simple, avec une variation linéaire du débit en fonction de la pression.

Les autres courbes sont obtenues en présence de 50 µg de particules magnétiques Dynabeads M-280. En augmentant la pression de 0 à 30 mbar à un taux de 1 mbar/min (courbe B), le débit reste inférieur au seuil de détection du débitmètre. Les particules magnétiques font donc obstruction au flux. Cela est confirmé par observation visuelle : on constate que le lit de particules magnétiques est dans un état compact, la pression étant insuffisante pour compenser les forces magnétiques en jeu. Puis, à 30 mbar environ, il y a un écoulement transitoire très rapide (courbe C), et à une pression supérieure, le débit retrouve la même valeur qu'en l'absence de particules magnétiques (courbe D).

Le système s'est donc comporté comme une vanne à seuil, qui permet l'écoulement seulement au-dessus d'une pression seuil.

L'observation visuelle montre qu'au moment du saut, toutes les particules quittent le canal : dans le dispositif particulier utilisé ici, quand on dépasse la pression capable de repousser contre le champ magnétique le bouchon compact de particules magnétiques, l'écoulement transitoire créé chasse les particules du canal.

La **figure 6B** illustre le comportement du système (débit en fonction de la pression) en présence de la conduite secondaire 13 présentant une résistance hydraulique élevée. A titre de conduite secondaire 13 (résistive), on utilise ici un tube en polyétheréthercétone (« PEEK ») de 65 µm de diamètre interne et de 8 cm de long. La résistance hydraulique de ce tube est d'environ 1,6 x 10¹⁴ Pa.s.m⁻³, alors que celle du canal 2 est d'environ 2,4 x 10¹³ Pa.s.m⁻³.

La courbe A est encore obtenue en l'absence de particules magnétiques. La courbe B est obtenue après introduction de 50 µg de particules Dynabeads M-280, en augmentant la pression à partir de 0 mbar, à un taux de 1 mbar/min. Jusqu'à 90 mbar environ, le débit reste inférieur au seuil de détection du débitmètre. Les particules magnétiques font donc obstruction au flux de par leur empilement compact (confirmé par observation visuelle).

Au-delà d'un seuil situé à 90 mbar environ, le lit de particules magnétiques se dilate, causant une augmentation du débit jusqu'à 3 µL/min environ (courbe C). Au-delà de ce seuil, le lit de particules magnétiques est dans un état fluidisé.

Contrairement au cas précédent, lorsque la pression est réduite sous le seuil de 90 mbar (courbe D), le débit diminue de façon progressive et affine. Cette portion de courbe peut être parcourue réversiblement. A l'observation, on constate que les particules magnétiques sont restées dans la zone de capture, et qu'aucune ne s'échappe.

Le système se comporte ici comme un système qui permet d'imposer une différence de pression constante et indépendante du débit, entre deux points d'un système microfluidique, ici l'entrée et la sortie de la zone de capture.

On note donc que l'utilisation d'une résistance hydraulique de sortie permet de faciliter la mise en oeuvre du système et de lui apporter de nouvelles fonctionnalités de contrôle d'écoulement ou de pression. Cependant, il faut noter que d'autres modes de réalisation, en particulier présentant des connections plus rigides, ou d'autres modes de contrôle d'écoulement, ou d'autres géométries de canal, peuvent ne pas nécessiter une telle résistance hydraulique *via* une conduite secondaire.

L'observation visuelle révèle que le volume apparent du domaine occupé par les particules magnétiques croît progressivement quand on augmente le débit. On observe également que ce domaine a une extension bien définie dans le canal 2, qui augmente avec le débit, et reste relativement homogène.

Cette découverte surprenante, et différente de tous les systèmes de l'état de l'art constitue un avantage majeur de l'invention. Elle partage en effet certains avantages des lits fluidisés, qui dans l'art antérieur nécessitaient l'utilisation de la gravité et des volumes beaucoup plus importants. Par ailleurs, du fait de la faiblesse de la force gravitationnelle sur des particules micrométriques, les lits fluidisés de l'état de l'art sont utilisables surtout avec des gaz, alors que dans l'invention on peut également travailler avec des liquides, qui exercent une friction hydrodynamique beaucoup plus grande.

En observation l'extension du domaine du canal 2 occupé par les particules magnétiques selon le débit, on peut calculer la porosité correspondante du lit de particules magnétiques.

Les résultats sont les suivants :
- Débit de 0 µL/min : porosité de 41 %.
- Débit de 0,5 µL/min : porosité de 66 %.
- Débit de 1 µL/min : porosité de 71 %.
- Débit de 1,5 µL/min : porosité de 75 %.
- Débit de 2 µL/min : porosité de 77 %.
- Débit de 2,5 µL/min : porosité de 79 %.
- Débit de 3 µL/min : porosité de 80 %.
- Débit de 3,5 µL/min : porosité de 81 %.
- Débit de 4 µL/min : porosité de 82 %.

A titre de comparaison, la porosité d'un système compact est généralement comprise entre 40 et 60% (pour des particules sphériques). On constate donc que l'invention offre un lit de particules magnétiques de configuration dense mais non compacte, ce qui est favorable pour optimiser à la fois le débit et l'échange entre les particules et le milieu.

### Exemple 3 - immunodosage par fluorescence

Une immunoextraction est effectuée au moyen de particules Dynabeads de 2,8 µm greffées avec des IgG de mouton ciblant des IgG de lapin (fournisseur : Invitrogen). Les expériences sont effectuées dans un tampon phosphate (PBS) contenant 0,1 % d'albumine de sérum bovin (BSA) pour limiter l'adsorption non-spécifique. Deux types d'IgG marqués fluorescents sont utilisés pour évaluer la spécificité du test : les anticorps IgG de lapin anti-souris Alexa Fluor® 488 (cible spécifique) et les anticorps IgG1 monoclonaux de souris anti-humains CD1a (cible non-spécifique). On utilise comme tampons d'élution un tampon citrate (0,1 M, pH 2-3), un tampon ammonium (0,16 % ammonium) à pH 9 et une solution de dodécylsulfate de sodium (SDS) à 100 mM.

On utilise le système microfluidique représenté sur la **figure 1**.

Le procédé d'immunoextraction nécessite l'injection séquentielle de différentes solutions dans le système avec un contrôle précis des volumes injectés en évitant le mélange des réactifs. On utilise un système MAESFLO (Fluigent) pour le contrôle des pressions, des vannes solénoïdes à action directe, et des tubes de PEEK entre les réservoirs et les entrées du système.

La première entrée 9 du canal d'alimentation 8 est reliée à un réservoir de tampon d'élution (tampon citrate, pH 2).

La deuxième entrée 10 du canal d'alimentation 8 est reliée à un réservoir d'échantillon (soit IgG de lapin anti-souris Alexa Fluor® 488 soit IgG1 monoclonaux de souris anti-humains CD1a).

La troisième entrée 11 du canal d'alimentation 8 est reliée à un réservoir de tampon de rinçage (PBS 1x, 0,1 % BSA, pH 7,4).

Les solutions sont injectées en appliquant une pression de 45 mbar au réservoir concerné avec le contrôleur de pressions. Le débit est mesuré avec un débitmètre intégré à la sortie du dispositif. Une fenêtre de détection de fluorescence est ménagée dans la conduite secondaire 13.

Les étapes de l'immunoextraction sont les suivantes :
- Rinçage des particules magnétiques.
- Incubation d'échantillon.
- Rinçage pour récupérer les biomolécules non liées.
- Elution des cibles isolées.

Avant tout test, le système microfluidique est revêtu avec un polymère époxy-diméthylacrylamide, afin d'éviter l'adsorption de particules et de protéines à la surface du canal. On injecte une solution de 0,15 % p/p du polymère juste après oxydation par plasma du canal. Le polymère est incubé pendant 30 minutes puis rincé avec le tampon de rinçage.

On pré-remplit le système avec le tampon de rinçage. La température est de 20°C pendant toute l'expérience.

La solution d'IgG marquée et le tampon d'élution sont mis en place dans leurs réservoirs.

Le piégeage initial des particules magnétiques est effectué en déconnectant la première entrée 9 du canal d'alimentation 8 du contrôleur et en appliquant une pointe de pipette pour injecter 5 µL de suspension de particules magnétiques. Les particules entrent dans le système par sédimentation et sont entraînées par le champ magnétique dans le canal 2. Puis la première entrée 9 est reconnectée au contrôleur de pression, et une pression est appliquée à la première entrée 9 et à la troisième entrée 11.

Une étape de rinçage est effectuée pour équilibrer l'immunosupport.

Puis l'échantillon de solution contenant l'IgG spécifique marqué (ou l'IgG de référence non-spécifique) est infusé à un débit de 1 µL/min (à 100 ng/mL). Les composés non liés sont ensuite éliminés avec un rinçage avec 20 µL de tampon de rinçage à 2 µL/min.

Puis on effectue une étape d'élution par injection de tampon d'élution.

Dans un premier mode d'élution, on injecte 20 µL de tampon à un débit de 0,5 µL/min.

Dans un deuxième mode d'élution, on injecte 2 µL de tampon à un débit de 0,5 µL/min (régime de lit fluidisé) puis on arrête le flux pendant 10 minutes, l'immunosupport étant alors en régime d'empilement compact. Cet arrêt laisse plus du temps à la dissociation, améliorant le rendement total de récupération de la cible. La compacité du lit permet aussi de limiter la dispersion de la cible et d'améliorer la sensibilité du test. Puis on réactive le flux pendant 2 minutes pour libérer les cibles.

On détecte la quantité d'IgG élué dans la fenêtre de détection, par mesure d'un signal de fluorescence.

Afin de faciliter le redémarrage de l'écoulement après la période d'arrêt, il est à noter qu'il est préférable de conserver le lit de particules dans un état compact, tout en maintenant entre l'entrée et la sortie de la chambre une différence de pression finie mais insuffisante pour sortir de l'état compact (P<PE dans la **figure 6B**).

Les performances de ces modes d'élution ont été comparées.

La **figure 7A** illustre le pic d'élution obtenu avec le mode d'élution continue (sur 50 min). Une large bande (6 min) est obtenue avec une hauteur de 100 u.a. et un rapport signal sur bruit d'environ 20.

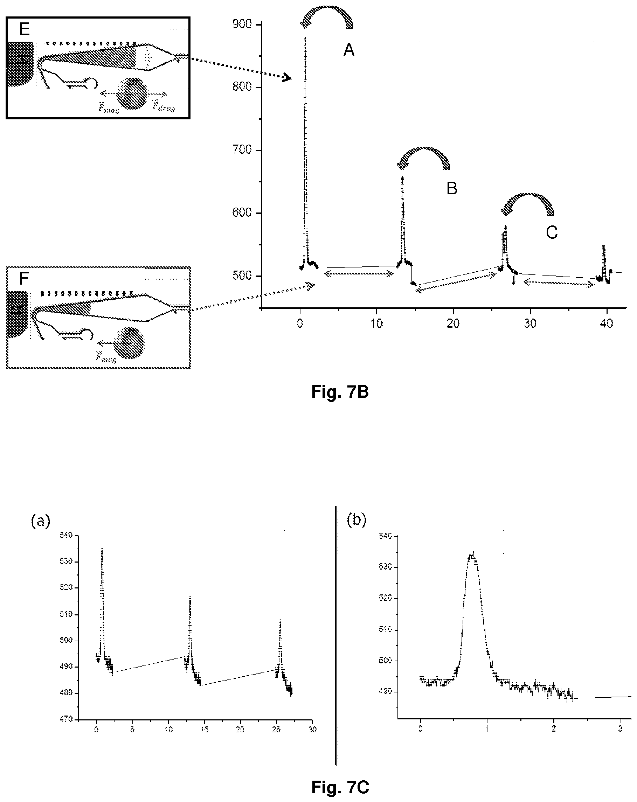

La **figure 7B** illustre les résultats avec le mode alterné. On effectue quatre élutions successives. A chaque fois le signal de fluorescence est mesuré pendant 2 minutes, avec un temps de latence de 10 minutes. Les pics d'élution sont plus marqués dans ce mode, avec une intensité décroissante avec le nombre d'élutions.

Le rapport signal sur bruit est amélioré d'un facteur de 3,5 environ pour la première élution par rapport au premier mode d'élution.

Sur la figure, le repère A correspond à la première élution, le repère B à la deuxième élution, le repère C à la troisième élution. Sur la gauche, on a représenté en E l'extension du lit de particules magnétiques dans le canal lorsque le fluide circule, et en F l'extension du lit de particules magnétiques dans le canal lorsque la circulation de fluide est stoppée. Les vecteurs F_{mag} et F_{drag} représentent la force magnétique et la force hydrodynamique exercées sur les particules.

La limite de détection est testée en injectant 20 µL de solution d'IgG marqué à 10 ng/mL, soit 0,2 ng d'IgG marqué. On utilise le mode d'élution alterné. On obtient un signal rapport sur bruit d'environ 7,5 et une limite de détection 3 ng/mL environ.

Enfin, en injectant 200 µL d'une solution à 6 pM, la cible est détectée avec un rapport signal sur bruit de fluorescence d'environ 10 : **figure 7C**. La partie (b) de la figure représente un détail de la partie (a). A titre de comparaison, dans l'état de la technique, avec des particules magnétiques similaires et des marqueurs similaires, la limite de détection était de 100 ng/mL : voir Mohamadi et al. dans Biomicrofluidics 5,044114 (2011).

### Exemple 4 - dispositif oscillant

On réalise une expérience similaire à celle de l'exemple 2, en fixant en plus un élément vibrant sur la tubulure de sortie. L'élément vibrant est un moteur rotatif (Précision Drive 4 mm, référence 304-101). Il est opéré à une tension de 3 V.

On étudie l'évolution du débit en fonction de la pression imposée. Les résultats sont visibles sur la **figure 8**. La courbe A correspond une mesure sans particules magnétiques. La courbe B correspond à une mesure avec particules magnétiques sans vibrations. Et la courbe C correspond à une mesure avec particules magnétiques et avec vibrations.

On constate que la présence des vibrations mécaniques, en luttant contre la tendance à l'agrégation des particules magnétiques, permet d'obtenir de diminuer la pression nécessaire pour obtenir un débit donné, ce qui augmente l'efficacité du système.

### Exemple 5 - capture sélective de bactéries de type salmonelles en présence de colibacilles

Un dispositif selon la **Figure 1** et l'exemple 1 est construit, et contrôlé par un système de contrôle en débit MAESFLO de la société Fluigent.

Le canal est tout d'abord chargé avec 50 µg de billes magnétiques de la même façon que dans l'exemple 3, excepté la nature des billes qui sont ici porteuses d'anticorps anti-salmonelles.

Une suspension contenant des salmonelles inactivées et marquées à l'aide d'un marqueur fluorescent vert, à la concentration de saturation divisée par 1000, dans un tampon phosphate addition de 0,1 % de BSA (albumine de sérum bovin), ainsi que des colibacilles *E. coli* à la même concentration et marqués par un marqueur fluorescent rouge, est perfusée dans la chambre contenant les billes magnétiques, à un débit de 1 µL/min pendant 20 minutes. Le système est ensuite rincé avec du tampon sans bactéries.

Après le rinçage, on effectue des photos du système dans les deux canaux de fluorescence rouge et vert. On évalue le rapport de capture comme le rapport du nombre de pixels présentant dans le vert une valeur supérieure à 3 fois le bruit de fond, au nombre de pixels présentant dans le rouge une valeur supérieure à 3 fois le bruit de fond. Ce rapport, qui est égal à 80 ± 10, montre la bonne spécificité du système. En outre, ce rapport est en réalité sous-évalué, car l'espèce la plus abondante capturée (les salmonelles) donnent plus fréquemment lieu à la superposition de plusieurs bactéries dans le même pixel, et donc à une sous-estimation du nombre de bactéries.

## Revendications

1. Système microfluidique (1) comprenant :
- au moins un canal (2) pour l'écoulement de fluide présentant une entrée (4), une sortie (5), et un axe longitudinal (7) s'étendant entre l'entrée (4) et la sortie (5), ledit canal (2) comprenant une zone de capture (3), et
- des moyens d'application (6) d'un champ magnétique présentant une intensité décroissante dans la zone de capture (3) du canal (2), de l'entrée (4) vers la sortie (5) du canal, **caractérisé en ce que** la section du canal (2) orthogonale à l'axe longitudinal (7) du canal (2) est croissante dans la zone de capture (3), de l'entrée (4) vers la sortie (5) du canal (2).

2. Système (1) selon la revendication 1, dans lequel le champ magnétique appliqué dans la zone de capture (3) est essentiellement parallèle à l'axe longitudinal (7) du canal (2).

3. Système (1) selon la revendication 1, dans lequel le champ magnétique appliqué dans la zone de capture (3) est essentiellement orthogonal à l'axe longitudinal (7) du canal (2).

4. Système selon l'une des revendications 1 à 3, dans lequel le champ magnétique décroit de manière continue d'une extrémité à l'autre de la zone de capture (3) de l'entrée (4) vers la sortie (5) du canal.

5. Système (1) selon l'une des revendications 1 à 4, dans lequel les moyens d'application (6) du champ magnétique comprennent ou consistent en un aimant disposé à l'extérieur du canal (2), de préférence du côté de l'entrée (4) de celui-ci.

6. Système (1) selon l'une des revendications 1 à 5, dans lequel la zone de capture (3) du canal (2) comprend des particules magnétiques, de préférence des particules super-paramagnétiques, la fraction volumique de ces particules dans la zone de capture (3) du canal (2) étant de préférence de 0,001 à 0,1 ou de 0,01 à 0,3 ; et la porosité du lit de particules valant de préférence de 50 à 95 %, de manière plus particulièrement préférée de 60 à 90 %.

7. Système (1) selon l'une des revendications 1 à 6, dans lequel la sortie (5) du canal (2) est connectée à une conduite secondaire (13) dont la résistance hydraulique est supérieure à la résistance hydraulique du canal (2), de préférence d'un facteur supérieur ou égal à 2, ou à 5 ou à 10.

8. Système (1) selon l'une des revendications 1 à 7, comprenant des moyens de déplacement de fluide pour effectuer l'écoulement de fluide de l'entrée vers la sortie du canal, et optionnellement un dispositif adapté à générer des oscillations de fluide dans le canal (2), de préférence choisi parmi les transducteurs soniques, ultrasoniques, ou piézoélectriques, les éléments vibrants, les haut-parleurs et les pistons oscillants.

9. Procédé de traitement d'un échantillon, comprenant une étape d'écoulement de fluide dans un canal (2) présentant une zone de capture (3), la vitesse d'écoulement du fluide étant décroissante dans la zone de capture (3), et un champ magnétique étant appliqué dans la zone de capture (3), présentant une intensité décroissante dans le sens d'écoulement du fluide.

10. Procédé selon la revendication 9, dans lequel, lors de l'étape d'écoulement de fluide, le champ magnétique appliqué est essentiellement parallèle à la direction moyenne d'écoulement du fluide dans la zone de capture (3).

11. Procédé selon la revendication 9, dans lequel, lors de l'étape d'écoulement de fluide, le champ magnétique appliqué est essentiellement orthogonal à la direction moyenne d'écoulement du fluide dans la zone de capture (3).

12. Procédé selon l'une des revendications 9 à 11, dans lequel, lors de l'étape d'écoulement de fluide, la zone de capture (3) contient des particules magnétiques, de préférence super-paramagnétiques, et dans lequel le fluide s'écoulant dans le canal (2) contient des analytes ; le procédé comprenant optionnellement une étape supplémentaire de détection d'analytes et / ou une étape supplémentaire de collecte d'analytes en aval du canal (2).

13. Procédé selon la revendication 12, comprenant au moins une étape au cours de laquelle les particules magnétiques au sein de la zone de capture (3) présentent au moins l'une des caractéristiques suivantes :
- les particules ne sont pas organisées de façon compacte, la fraction volumique de ces particules dans la zone de capture (3) du canal (2) valant de préférence de 0,01 à 0,3, ou la porosité du lit de particules valant de 50 à 95 %, de préférence de 60 à 90 % ;
- les particules sont constituées en lit fluidisé ;
- les particules sont réparties de manière homogène au sein de la zone de capture (3) ;
- les particules subissent une recirculation continue lors de l'écoulement du fluide.

14. Procédé selon la revendication 13, comprenant au moins une autre étape au cours de laquelle les particules magnétiques au sein de la zone de capture (3) sont organisées en lit compact, la fraction volumique des dites particules magnétiques valant de préférence de 0,4 à 0,6.

15. Procédé selon l'une des revendications 9 à 14, comprenant la mise en oeuvre d'une réaction et / ou séparation chimique, biochimique et / ou biologique, de préférence choisie parmi les réactions catalytiques, les hybridations, les réactions électrochimiques, les réactions enzymatiques, les immunodosages, les séparations chromatographiques, les réactions de chimioluminescence, les captures immunologiques, les captures par affinité, les élutions, les purifications, les concentrations, les extractions et combinaisons de celles-ci.

## Patentansprüche

1. Mikrofluidisches System (2), umfassend:
- mindestens einen Kanal (2) zum Abfließen von Fluid, aufweisend einen Eingang (4), einen Ausgang (5) und eine Längsachse (7), die sich zwischen dem Eingang (4) und dem Ausgang (5) erstreckt, wobei der Kanal (2) einen Erfassungsbereich (3) umfasst,
- Mittel zum Anwenden (6) eines Magnetfelds, das eine abnehmende Stärke im Erfassungsbereich (3) des Kanals (2) vom Eingang (4) hin zum Ausgang (5) des Kanals umfasst, **dadurch gekennzeichnet, dass** der Abschnitt des Kanals (2) orthogonal zur Längsachse (7) des Kanals (2) im Erfassungsbereich (3) vom Eingang (4) hin zum Ausgang (5) des Kanals (2) ansteigend ist.

2. System (1) nach Anspruch 1, wobei das Magnetfeld, angewendet im Erfassungsbereich (3), im Wesentlichen parallel zur Längsachse (7) des Kanals (2) ist.

3. System (1) nach Anspruch 1, wobei das Magnetfeld, angewendet im Erfassungsbereich (3), im Wesentlichen orthogonal zur Längsachse (7) des Kanals (2) ist.

4. System nach einem der Ansprüche 1 bis 3, wobei das Magnetfeld auf kontinuierliche Weise von einem Ende zum anderen des Erfassungsbereichs (3) vom Eingang (4) hin zum Ausgang (5) des Kanals abnimmt.

5. System (1) nach einem der Ansprüche 1 bis 4, wobei die Mittel zum Anwenden (6) des Magnetfelds einen Magneten umfassen oder aus diesem bestehen, der an außerhalb des Kanals (2), vorzugsweise auf der Seite des Eingangs (4) desselben angeordnet ist.

6. System (1) nach einem der Ansprüche 1 bis 5, wobei der Erfassungsbereich (3) des Kanals (2) magnetische Partikel umfasst, vorzugsweise superparamagnetische Partikel, wobei die Volumenfraktion dieser Partikel im Erfassungsbereich (3) des Kanals (2) vorzugsweise von 0,001 bis 0,1 oder von 0,01 bis 0,3 ist; und die Porosität des Partikelbetts vorzugsweise einen Wert von 50 bis 95 % aufweist, insbesondere vorzugsweise von 60 bis 90 %.

7. System (1) nach einem der Ansprüche 1 bis 6, wobei der Ausgang (5) des Kanals (2) mit einer sekundären Leitung (13) verbunden ist, deren hydraulischer Widerstand höher als der hydraulische Widerstand des Kanals (2) ist, vorzugsweise um einen Faktor von mehr als oder gleich 2 oder 5 oder 10.

8. System (1) nach einem der Ansprüche 1 bis 7, umfassend Mittel zum Verschieben von Fluid um das Abfließen von Fluid vom Eingang hin zum Ausgang des Kanals durchzuführen, und optional eine Vorrichtung, die ausgelegt ist, um Oszillationen von Fluid im Kanal (2) zu erzeugen, vorzugsweise ausgewählt aus den Schall-, Ultraschall-, oder piezoelektrischen Wandlern, vibrierenden Elementen, Lautsprechern und oszillierenden Kolben.

9. Verfahren zur Behandlung einer Probe, umfassend einen Schritt des Abfließens von Fluid in einem Kanal (2), der einen Erfassungsbereich (3) aufweist, wobei die Abfließgeschwindigkeit des Fluids im Erfassungsbereich (3) abnehmend ist, und wobei ein Magnetfeld im Erfassungsbereich (3) angewendet wird, das eine abnehmende Stärke in der Richtung des Abfließens des Fluids aufweist.

10. Verfahren nach Anspruch 9, wobei, beim Schritt des Abfließens von Fluid, das angewendete Magnetfeld im Wesentlichen parallel zur mittleren Richtung des Abfließens des Fluids im Erfassungsbereich (3) ist.

11. Verfahren nach Anspruch 9, wobei, beim Schritt des Abfließens von Fluid, das angewendete Magnetfeld im Wesentlichen orthogonal zur mittleren Richtung des Abfließens des Fluids im Erfassungsbereich (3) ist.

12. Verfahren nach einem der Ansprüche 9 bis 11, wobei beim Schritt des Abfließens von Fluid der Erfassungsbereich (3) magnetische, vorzugsweise superparamagnetische Partikel umfasst, und wobei das Fluid, das im Kanal (2) abfließt, Analyten umfasst; wobei das Verfahren optional einen zusätzlichen Schritt des Nachweisens von Analyten und/oder einen zusätzlichen Schritt des Sammelns von Analyten nachgelagert vom Kanals (2) umfasst.

13. Verfahren nach Anspruch 12, umfassend mindestens einen Schritt, im Laufe dessen die magnetischen Partikel im Inneren des Erfassungsbereichs (3) mindestens eines der folgenden Merkmale aufweisen:
- die Partikel sind nicht auf kompakte Weise organisiert, wobei die Volumenfraktion dieser Partikel im Erfassungsbereich (3) des Kanals (2) vorzugsweise einen Wert von 0,01 bis 0,3 aufweist oder die Porosität des Partikelbetts einen Wert von 50 bis 95 %, vorzugsweise von 60 bis 90 % aufweist;
- die Partikel sind in einer Wirbelschicht gebildet;
- die Partikel sind auf homogene Weise im Inneren des Erfassungsbereichs (3) verteilt;
- die Partikel sind einer kontinuierlichen Rezirkulation beim Abfließen des Fluids unterzogen.

14. Verfahren nach Anspruch 13, umfassend mindestens einen weiteren Schritt, im Verlaufe dessen die magnetischen Partikel im Inneren des Erfassungsbereichs (3) in einem kompakten Bett organisiert sind, wobei die Volumenfraktion der magnetischen Partikel vorzugsweise einen Wert von 0,4 bis 0,6 aufweist.

15. Verfahren nach einem der Ansprüche 9 bis 14, umfassend die Durchführung einer chemischen, biochemischen und/oder biologischen Reaktion und/oder Trennung, vorzugsweise ausgewählt aus den katalytischen Reaktionen, Hybridisierungen, elektrochemischen Reaktionen, enzymatischen Reaktionen, Immunoessays, chromatographischen Trennungen, Chemolumineszenzreaktionen, Immunerfassungen, Erfassungen durch Affinität, Elutionen, Reinigungen, Konzentrationen, Extraktionen und Kombinationen derselben.

## Claims

1. Microfluidic system (1) comprising:
- at least one channel (2) for the flow of fluid having an inlet (4), an outlet (5) and a longitudinal axis (7) extending between the inlet (4) and the outlet (5), said channel (2) comprising a capture zone (3), and
- means (6) for applying a magnetic field having a decreasing intensity in the capture zone (3) of the channel (2), from the inlet (4) towards the outlet (5) of the channel, **characterized in that** the cross-sectional area of the channel (2) orthogonal to the longitudinal axis (7) of the channel (2) increases in the capture zone (3), from the inlet (4) towards the outlet (5) of the channel (2).

2. System (1) according to claim 1, wherein the magnetic field applied in the capture zone (3) is essentially parallel to the longitudinal axis (7) of the channel (2).

3. System (1) according to claim 1, wherein the magnetic field applied in the capture zone (3) is essentially orthogonal to the longitudinal axis (7) of the channel (2).

4. System according to any one of claims 1 to 3, wherein the magnetic field continuously decreases from one extremity of the capture zone (3) to the other from the inlet (4) towards the outlet (5) of the channel.

5. System (1) according to any one of claims 1 to 4, wherein the means (6) for applying the magnetic field comprise or consist of a magnet disposed outside the channel (2), preferably on the side of the inlet (4) thereof.

6. System (1) according to any of claims 1 to 5, wherein the capture zone (3) of the channel (2) comprises magnetic particles, preferably superparamagnetic particles, the volume fraction of these particles in the capture zone (3) of the channel (2) preferably being from 0.001 to 0.1 or from 0.01 to 0.3; and the porosity of the bed of particles ranging preferably from 50 to 95 %, more particularly preferably from 60 to90 %.

7. System (1) according to any of claims 1 to 6, wherein the outlet (5) of the channel (2) is connected to a secondary conduit (13), the hydraulic resistance of which is greater than the hydraulic resistance of the channel (2), preferably by a factor greater than or equal to 2, or 5, or 10.

8. System (1) according to any of claims 1 to 7, comprising means for moving fluid in order to effect the flow of fluid from the inlet towards the outlet of the channel, and optionally a device adapted for generating oscillations of fluid in the channel (2), preferably chosen from sonic, ultrasonic or piezoelectric transducers, vibrating elements, loudspeakers and oscillating pistons.

9. Method of processing a sample, comprising a step of fluid flowing in a channel (2) having a capture zone (3), the speed of flow of the fluid decreasing in the capture zone (3), and a magnetic field being applied in the capture zone (3), having an intensity decreasing in the direction of flow of the fluid.

10. Method according to claim 9, wherein, during the step of fluid flowing, the magnetic field applied is essentially parallel to the average direction of flow of the fluid in the capture zone (3).

11. Method according to claim 9, wherein, during the step of fluid flowing, the magnetic field applied is essentially orthogonal to the average direction of flow of the fluid in the capture zone (3).

12. Method according to any one of claims 9 to 11, wherein, during the step of fluid flowing, the capture zone (3) contains magnetic, preferably superparamagnetic, particles, and wherein the fluid flowing in the channel (2) contains analytes; the method optionally comprising a supplementary step of detecting analytes and/or a supplementary step of collecting analytes downstream of the channel (2).

13. Method according to claim 12, comprising at least one step during which the magnetic particles within the capture zone (3) present at least one of the following features:
- the particles are not organized in a compact way, the volume fraction of these particles in the capture area (3) of the channel (2) ranging preferably from 0.01 to 0.3; or the porosity of the bed of particles ranging preferably from 50 to 95 %, preferably from 60 to90 %.
- the particles are constituted in a fluidized bed;
- the particles are distributed homogeneously within the capture zone (3);
- the particles undergo a continuous recirculation during the fluid flowing.

14. Method according to claim 13, comprising at least another step during which the magnetic particles are organized in a compact bed, the volume fraction of said magnetic particles ranging preferably from 0.4 to 0.6.

15. Method according to any of claims 9 to 14, comprising the implementation of a chemical, biochemical and/or biological reaction and/or separation, preferably chosen from catalytic reactions, hybridisations, electrochemical reactions, enzymatic reactions, immunoassays, chromatographic separations, chemiluminescence reactions, immunological captures, affinity captures, elutions, purifications, concentrations, extractions and combinations thereof.
